# EUROPEAN PATENT SPECIFICATION

(11) **EP 2 658 605 B1**
(45) Date of publication and mention of the grant of the patent: **27.07.2016**
(21) Application number: 11811486.7
(22) Date of filing: 28.12.2011
(51) Int. Cl.: A61N 1/362, A61N 1/365

(54) **ENDOVASCULAR CAROTID BODY ABLATION CATHETER FOR SYMPATHETIC REBALANCING OF PATIENT**
ENDOVASKULARER HALSSCHLAGKÖRPER-ABLATIONSKATHETER ZUR SYMPATHISCHEN REKOMPENSATION VON PATIENTEN
CATHETER D'ABLATION ENDOVASCULAIRE DU CORPUSCULE CAROTIDIEN POUR LE RÉÉQUILIBRAGE SYMPATHIQUE D'UN PATIENT

(30) Priority: 29.06.2011 US 201161502377 P; 28.12.2010 US 201061427657 P
(43) Date of publication of application: 06.11.2013
(73) Proprietor: Cibiem, Inc., Los Altos, CA 94022 (US)
(72) Inventor: GELFAND, Mark, Los Altos CA 94022 (US); LEVIN, Howard, Los Altos CA 94022 (US)
(74) Representative: Peterreins Schley
(86) International application number: PCT/US2011/067568
(87) International publication number: WO 2012/092364

(56) References cited:
- WO-A1-97/25916
- US-A- 5 476 495
- US-A1- 2002 128 639
- US-A1- 2003 216 792
- US-A1- 2005 288 730
- US-A1- 2006 253 161
- US-A1- 2006 259 084
- US-A1- 2008 009 917
- US-A1- 2010 070 004
- US-A1- 2010 274 219

## Description

### TECHNICAL FIELD

The present disclosure is directed generally to systems for treating patients having cardiac, metabolic, and pulmonary disease associated at least in part with peripheral chemoreceptor hypersensitivity or heightened sympathetic activation by ablating at least one peripheral chemoreceptor (e.g. carotid body).

### BACKGROUND

It is known that an imbalance of the autonomic nervous system is associated with several disease states. Restoration of autonomic balance has been a target of several medical treatments including modalities such as pharmacological, device-based, and electrical stimulation. For example, beta blockers are a class of drugs used to reduce sympathetic activity to treat cardiac arrhythmias and hypertension; Gelfand and Levin (US 7,162,303) describe a device-based treatment used to decrease renal sympathetic activity to treat heart failure, hypertension, and renal failure; Yun and Yuarn-Bor (US 7,149,574; US 7,363,076; US 7,738,952) describe a method of restoring autonomic balance by increasing parasympathetic activity to treat disease associated with parasympathetic attrition; Kieval, Burns and Serdar (US 8,060,206) describe an electrical pulse generator that stimulates a baroreceptor, increasing parasympathetic activity, in response to high blood pressure; Hlavka and Elliott (US 2010/0070004) describe an implantable electrical stimulator in communication with an afferent neural pathway of a carotid body chemoreceptor to control dyspnea via electrical neuromodulation.

### SUMMARY OF THE INVENTION

Many congestive heart failure (CHF) and hypertension (HTN) patients with intense regimens of state of the art drugs, and yet resisting therapy, exhibit significant hypersensitivity or hyperactivity of peripheral chemosensors. The inventors conclude that this elevated peripheral chemosensitivity or hyperactivity contributes to sustaining autonomic imbalance and disease state and further disease progression.

Devices have been conceived by the inventors that reduce chemosensitivity of a patient by directly reducing, inhibiting, or removing input from at least one peripheral chemoreceptor using surgical techniques or minimally invasive interventional techniques such as, for example, endovascular catheterization or percutaneous puncture guided and assisted by MRI, sonography, CT or fluoroscopy. The inventors have conceived methods of treating sympathetically mediated disease, in which enhanced sensitivity of peripheral chemoreceptors is a contributor to pathology, and restore autonomic balance via modulation of peripheral chemoreflex via ablation of chemosensitive cells (also historically called glomus cells), related afferent nerve endings, or related adjacent structures in the carotid body or aortic body. It is expected that such interventions may lead to relief of the sensation of breathlessness, reduction of hyperventilation, better exercise capacity, vasodilation, reduction of sympathetic activation, reduction of fluid retention, improved glucose metabolism, or reduction of hypertension.

The methods of the present disclosure may include surgical modulation of a peripheral chemoreceptor. Surgical techniques may involve direct or endoscopic access to a peripheral chemoreceptor through an incision in the patient's skin proximate to the peripheral chemoreceptor. Stimulation may optionally be used to confirm identification of a chemoreceptor prior to modulation. Stimulation can also be used after the procedure to confirm success.

Alternatively, the methods of the present disclosure may be minimally invasive with less risk than is associated with surgery. The procedure may take as little time as possible so as not to overuse expensive hospital resources and not subject a patient to additional risk of deep anesthesia. A method has been conceived to screen for treatment a patient suffering a sympathetically mediated disease, the method comprising: determining whether the patient has a sympathetic tone higher than a threshold sympathetic tone; assessing the activity of the carotid body in the patient, and based on the assessment of the activity of the carotid body and the determination that the patient has a high sympathetic tone, determining whether the patient is a candidate for the treatment.

A method has been conceived to assess a patient for a treatment to suppress activity of the carotid body in the patient, the method comprising: selecting the patient based on a diagnosis that the patient suffers from a sympathetically mediated disease; determining that the patient has a sympathetic tone higher than a threshold sympathetic tone; assessing the activity of the carotid body in the patient; based on the assessment of the activity of the carotid body and the determination that the patient has a high sympathetic tone, determining that the patient is a candidate for the treatment; after the treatment determining whether the activity of the carotid body is below the assessed activity of the carotid body prior to the treatment, and determining if the lowering of the carotid body activity achieved a therapeutic effect with respect to the sympathetically mediated disease. The assessment of the activity of the carotid body may include stimulating a tissue of the patient and monitoring the response of the patient to the stimulation, and the step of determining whether the activity of the carotid body is below the assessed activity includes repeating the stimulation of the tissue and comparing the responses to the stimulations.

A method of treatment has been conceived comprising: assessing the activity of a carotid body in a patient; determining to suppress the activity of the carotid body of the patient based on assessed activity; suppressing the carotid body by accessing a region proximate to the carotid body in the patient, and determining whether the suppression of the activity of the carotid body achieved a therapeutic effect in the patient.

The suppression of the carotid body may include ablating the carotid body. The ablation of the carotid body may include performing an endovascular procedure comprising: connecting a proximal region of a catheter to an energy source; positioning a distal region of the catheter into a carotid artery of the patient; applying energy from the energy source to the proximal region of the catheter which conveys the energy to the distal region; delivering energy from the distal region of the catheter to an area of carotid artery bifurcation in the carotid artery, wherein the delivery of energy ablates the carotid body or carotid body nerves, and withdrawing the catheter from the carotid artery and from the patient after the ablation.

The positioning of the distal region of the catheter in the carotid artery may include: applying a low level of energy from the energy source to the proximal region of the catheter and conveying the energy to the distal region; delivering the low level of energy from the distal region to the carotid artery; detecting a response to the low level of energy by a nerve proximate to the carotid artery, and repositioning the distal region in the carotid artery, if the detected response is from a nerve other than the carotid body. The positioning of the catheter may also include: stimulating a tissue of the patient to provoke a physiological response by the patient; monitoring the physiological response to the stimulation, and based on the response, determining whether the distal region is properly positioned in the carotid artery.

A method has been conceived to treat a patient having sympathetically mediated disease comprising: diagnosing a patient as suffering from a sympathetically mediated disease; screening the diagnosed patient based on whether the patient has a chemosensitivity exceeding a threshold level of chemosensitivity; if the chemosensitivity exceeds the threshold level, selecting the patient for suppression of activity of a carotid body in the diagnosed patient and excluding the patient from selection if the chemosensitivity is below the threshold level; suppressing the activity of the carotid body of the selected patient, and determining whether the suppression of the carotid body achieved a therapeutic effect by reducing the suffering of the patient due to the sympathetically mediated disease.

A method has been conceived to treat cardiac, metabolic, and pulmonary diseases including: positioning an ablation device in a vascular system of a mammalian patient, wherein an ablating region of the ablation device is in a carotid artery of the vascular system and proximate to a carotid body of the mammalian patient; forming a lesion on tissue at or proximate to the carotid body by ablating the tissue using the ablation device; suppressing a chemoreflex function of the carotid body due to the lesion, and removing the ablation device from the patient.

A method has been conceived comprising: applying energy to mammalian tissue at or proximate to a carotid body of a mammalian patient, wherein the energy is applied by a distal region of a catheter inserted in a vascular system of the patient and the energy is supplied from an energy source external to the patient; ablating the tissue at or proximate to the carotid body due to the application of the energy; forming a lesion at the tissue due to the ablation of the tissue; removing the catheter from the patient, and suppressing or inhibiting natural carotid body chemoreflex functions due to the lesion and after removal of the catheter.

A method has been conceived to inhibit chemoreflex function generated by a carotid body in a mammalian patient to treat at least one of heart failure and hypertension, the method comprising: positioning an ablation device in a vascular system of the patient such that a distal section of the ablation device is proximate to or at the carotid body of the patient; supplying energy to a distal section of the ablation device wherein the energy is supplied by an energy supply apparatus outside of the patient; applying the energy from the energy supply through the ablation device to the distal section to ablate tissue proximate to or included in the carotid body; forming a lesion at the tissue due to the ablation of the tissue; removing the ablation device from the patient, and inhibiting carotid body chemoreflex functions due to the lesion and subsequent to the removal of the distal section.

A method has been conceived to treat a sympathetically mediated disease comprising: identifying a patient having enhanced sensitivity of chemoreceptors; directly reducing, inhibiting or removing output from at least one peripheral chemoreceptor using a surgical technique or a minimally invasive interventional technique, and the direct reduction, inhibiting or removal achieves an autonomic balance via modulation of peripheral chemoreflex.

The above described methods also may strive to preserve carotid sinus baroreflex.

It may be desired to identify patients most likely to benefit from therapy before the procedure is performed since generic pools of HTN and CHF patients are vast and diverse. It may also be desired to assess and quantitatively describe the results of the procedure in order to give a physician feedback on degree of success. In some cases a repeat procedure or bilateral procedure can be performed based on data from a unilateral procedure, for example. Non-invasive, relatively simple techniques to measure chemosensitivity or peripheral chemoreflex may be useful tools in the inventive therapy for identifying patients most likely to benefit or assess degree of success of a procedure.

The systems disclosed herein may be applied to satisfy clinical needs related to treating cardiac, metabolic, and pulmonary diseases associated, at least in part, with enhanced chemoreflex (e.g. high chemosensor sensitivity or high chemosensor activity) and related sympathetic activation. The present invention is expected to restore autonomic balance by reducing sympathetic activity, as opposed to increasing parasympathetic activity. It is understood that parasympathetic activity can increase as a result of the reduction of sympathetic activity (e.g. sympathetic withdrawal) and normalization of autonomic balance. Furthermore, the invention reduces sympathetic activity by modulating a peripheral chemoreflex. Enhanced peripheral and central chemoreflex is implicated in several pathologies including hypertension, cardiac tachyarrhythmias, sleep apnea, dyspnea, chronic obstructive pulmonary disease (COPD), diabetes and insulin resistance, and CHF. Central sympathetic nervous system activation is common to all these progressive and debilitating diseases. Peripheral chemoreflex may be modulated, for example, by modulating carotid body activity. The carotid body is the sensing element of the afferent limb of the peripheral chemoreflex. Carotid body activity may be modulated, for example, by ablating a carotid body or afferent nerves emerging from the carotid body. Therefore, a therapeutic method of the present disclosure comprises restoring autonomic balance by reducing or removing carotid body input into the central nervous system.

A method has been conceived to treat cardiac, metabolic, and pulmonary diseases including: positioning an ablation device in a vascular system of a mammalian patient, such that an ablating region of the ablation device is in the vascular system and proximate to a carotid body or carotid body afferent nerves of a mammalian patient; activating the ablation device while the ablating region is positioned proximate to the carotid body, and at least partially disabling carotid body chemoreflex functions as a result of the activation of the ablation device. The ablation device may include a catheter and the ablating region of the ablation device is at a distal end of the catheter, wherein the positioning includes positioning the distal end proximate to a carotid artery of the patient.

The method may further comprise: determining a baseline peripheral chemosensitivity of the mammalian patient, and performing the positioning step if the determined baseline peripheral chemosensitivity indicates the patient has a high chemosensitivity.

The method may further comprise: stimulating the carotid body to elicit a physiological response used to confirm said proximate position or relative proximity. For example, electric stimulation can confirm a distance of zero to 2-3 mm from excitable nerve tissue. A closer proximity generally elicits a stronger response. Such a method of application of electrical stimulation at one or more positions and measuring responses is called "mapping" of chemoreceptors. A sympathetic physiological response may be ventilation increase, blood pressure increase, blood flow increase, heart rate increase, and muscle sympathetic nerve activity increase. A parasympathetic response (e.g. decrease of ventilation, blood pressure, heart rate, blood flow, or muscle sympathetic nerve activity) can indicate undesired proximity to parasympathetic nerve fibers. Other undesired nerve stimulation responses may be observed (e.g. stimulation of a hypoglossal nerve may produce a response of protrusion of the tongue, stimulation of the sympathetic trunk may produce clinical signs of sympathetic activation).

The activation of the ablation device may include applying energy, e.g., thermal energy, to the ablating region to ablate tissue at or near the carotid body. The energy may be electrical energy applied as radio frequency (RF) energy and may be applied for less than three minutes, for example, less than one minute. The ablation of the tissue may form a lesion on the tissue, wherein the lesion suppresses or inhibits natural carotid body chemoreflex functions. The ablation may be significantly contained to a target region and may safely include a small margin proximate to the target region while not ablating baroreceptors or nerves of the neck unrelated to chemoreflex.

A method has been conceived comprising: applying energy to mammalian tissue at or proximate to the carotid body of a mammalian patient, wherein the energy is applied by a distal region of a catheter inserted in a vascular system of the patient and the energy is supplied from an energy source external to the patient; ablating the tissue at or proximate to the carotid body due to the application of the energy; removing the catheter from the patient, and suppressing or inhibiting natural carotid body chemoreflex functions as a result of the ablation of the tissue. The method may further comprise: determining a baseline peripheral chemosensitivity of the mammalian patient, and performing the positioning step if the determined baseline peripheral chemosensitivity indicates the patient has a high chemosensitivity.

A method has been conceived to inhibit chemoreflex function generated by a carotid body in a mammalian patient to treat at least one of heart failure and hypertension, the method comprising: positioning an ablation device in a vascular system of the patient such that a distal section of the ablation device can apply energy to tissue of the patient proximate to or at the carotid body of the patient; supplying energy to a distal section of the ablation device wherein the energy is supplied by an energy supply apparatus outside of the patient; applying the energy from the energy supply through the ablation, device to the distal section to ablate the tissue; removing the ablation device from the patient, and inhibiting carotid body chemoreflex functions due to the ablation and subsequent to the removal of the distal section.

A method has been conceived for reducing abnormally elevated chemoreflex signals to treat at least one of elevated sympathetic nerve activity, hyperventilation, heart failure, periodic breathing, dyspnea, insulin resistance, and hypertension, the method comprising: positioning an ablation device in a vascular system of the patient such that a distal section of the ablation device can apply energy to tissue of the patient proximate to or at the carotid body of the patient; supplying energy to a proximal section of the ablation device wherein the energy is supplied by an energy supply apparatus outside of the patient; applying the energy from the energy supply through the ablation device to the distal section to ablate the tissue; removing the ablation device from the patient, and inhibiting carotid body chemoreflex functions due to the application of the energy and subsequent to the removal of the distal section. The method may further comprise determining that the mammalian patient has abnormally elevated chemoreflex before the position of the ablation device. The method may further comprise testing changes of chemoreflex during the procedure, after the procedure, or after the removal of ablation device from the body.

### BRIEF DESCRIPTION OF THE DRAWINGS

FIGURE 1 is a cutaway illustration of vasculature and neural structures of a right side of a patient's neck.
FIGURE 2 to 4 are illustrations of surgical access to a patient's left carotid body.
FIGURE 5 is an illustration of a patient's right carotid arteries and bifurcation with a schematic view of an endovascular catheter inserted into the vasculature to ablate a carotid body.
FIGURE 6 is a schematic view of an endovascular ablation device ablating a carotid body along with a thermal protection catheter placed proximate a carotid sinus.
FIGURE 7 is a schematic view of an endovascular radiofrequency catheter ablating a carotid body.
FIGURE 8 is a schematic view of an endovascular bipolar radiofrequency catheter ablating a carotid body.
FIGURE 9 is a schematic view of an endovascular cooled-radiofrequency catheter ablating a carotid body.
FIGURE 10 is a schematic view of an endovascular catheter used for transvascular ablation of a carotid body.
FIGURE 11 is a schematic view of an endovascular catheter used for cryogenic ablation of a carotid body.
FIGURE 12 is a schematic view of an occlusion device used to embolize blood supply to a carotid body.
FIGURE 13 is a schematic view of a segregation catheter used to deliver an agent that may be used to visualize, chemically ablate, or embolize a carotid body.
FIGURE 14 is a schematic view of a percutaneous ablation device ablating a carotid body.
FIGURE 15 is a schematic view of a percutaneous ablation device ablating a carotid body along with a fiduciary endovascular catheter.
FIGURE 16 is a schematic view of a percutaneous ablation device ablating a carotid body along with a fiduciary endovascular catheter.
FIGURE 17 is a schematic view of a percutaneous ablation device ablating a carotid body aided with ultrasound imaging.
FIGURE 18 is a schematic view of a percutaneous ablation device ablating a carotid body aided with ultrasound imaging.
FIGURE 19 is a schematic view of a High Intensity Focused Ultrasound (HIFU) device ablating a carotid body.
FIGURE 20 is a computer tomography image of a patient's carotid artery showing a carotid body.
FIGURE 21 is a flow chart of a method for treating a patient involving assessing the patients's chemosensitivity as a selection criterion for a carotid body ablation procedure.
FIGURE 22 is a flow chart of a method for treating a patient involving assessing the patient's chemosensitivity and response to a temporary carotid body block as a selection criterion for a carotid body ablation procedure.

### DETAILED DESCRIPTION

The present invention is disclosed by the appended claims and directed generally to systems, and devices to ablate fully or partially one or both carotid bodies or carotid body chemoreceptors to treat patients having a cardiac, metabolic, and pulmonary disease (e.g. hypertension, CHF, diabetes) resulting from peripheral chemoreceptor hypersensitivity or heightened sympathetic activation. A reduction of peripheral chemosensitivity or reduction of afferent nerve signaling from the carotid body (CB) resulting in a reduction of central sympathetic tone is the main therapy pathway. Other important benefits such as reduction of dyspnea, hyperventilation and breathing rate may be expected in some patients. The term "ablate" may refer to the act of altering a tissue to suppress or inhibit its biological function or ability to respond to stimulation. For example, ablation may involve, but is not limited to, thermal necrosis (e.g. using energy such as thermal energy, radiofrequency electrical current, direct current, microwave, ultrasound, high intensity focused ultrasound, and laser), cryogenic ablation, electroporation, selective denervation (e.g. destruction of afferent nerves from the carotid body while preserving nerves from the carotid sinus which conduct baroreceptor signals), embolization (e.g. occlusion of blood vessels feeding the gland), artificial sclerosing of blood vessels, mechanical impingement or crushing, surgical removal, chemical ablation, or application of radiation causing controlled necrosis (e.g. brachytherapy). The treatment may involve inserting a catheter in the patient's vascular system, positioning an energy delivery element at the distal end of the catheter proximate to chemoreceptors and delivering ablative thermal energy to the chemoreceptors in order to ablate them. Other methods and devices for chemoreceptor ablation are described. Documents US-A1-2003/216972, WO-A1-97/25916, US-A1-2002/128639, and US-A-5 476 495 disclose different endovascular ablation catheters.

. Therapy Example: Role of Chemoreflex and Central Sympathetic Nerve Activity in CHF

Chronic elevation in sympathetic nerve activity (SNA) is associated with the development and progression of certain types of hypertension and contributes to the progression of congestive heart failure (CHF). It is also known that sympathetic excitatory cardiac, somatic, and central/peripheral chemoreceptor reflexes are abnormally enhanced in CHF and hypertension (Ponikowski, 2011 and Giannoni, 2008 and 2009).

Arterial chemoreceptors serve an important regulatory role in the control of alveolar ventilation. They also exert a powerful influence on cardiovascular function.

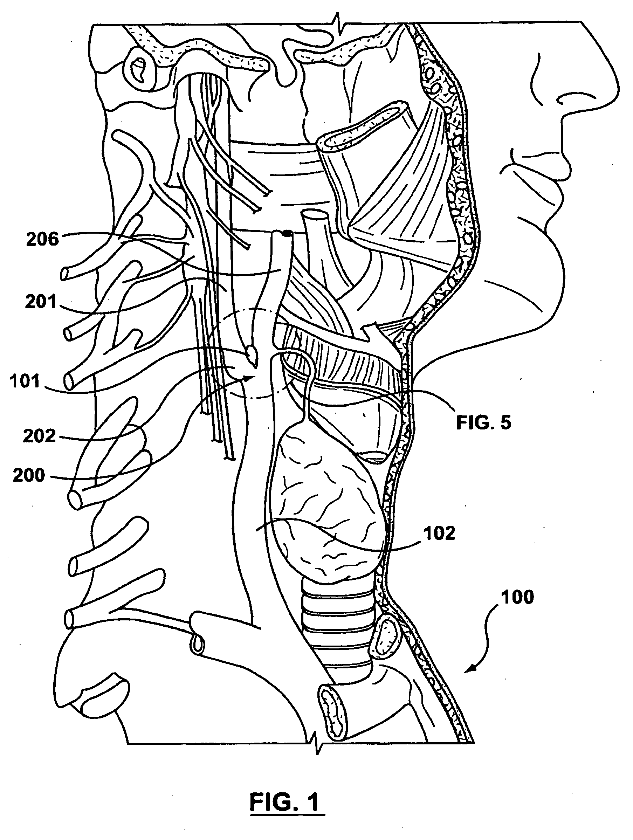

Delivery of Oxygen (O₂) and removal of Carbon Dioxide (CO₂) in the human body is regulated by two control systems, behavioral control and metabolic control. The metabolic ventilatory control system drives our breathing at rest and ensures optimal cellular homeostasis with respect to pH, partial pressure of carbon dioxide (PCO₂), and partial pressure of oxygen (PO₂). Metabolic control uses two sets of chemoreceptors that provide a fine-tuning function: the central chemoreceptors located in the ventral medulla of the brain and the peripheral chemoreceptors such as the aortic chemoreceptors and the carotid body chemoreceptors. As shown in FIGURE 1, the carotid body 101, a small, ovoid-shaped (often described as a grain of rice), and highly vascularized organ is situated in or near the carotid bifurcation 200, where the common carotid artery 102 branches in to an internal carotid artery (IC) 201 and external carotid artery (EC) 206. The central chemoreceptors are sensitive to hypercapnia (high PCO₂), and the peripheral chemoreceptors are sensitive to hypercapnia and hypoxia (low blood PO₂). Under normal conditions activation of the sensors by their respective stimuli results in quick ventilatory responses aimed at the restoration of cellular homeostasis.

As early as 1868, Pflüger recognized that hypoxia stimulated ventilation, which spurred a search for the location of oxygen-sensitive receptors both within the brain and at various sites in the peripheral circulation. When Corneille Heymans and his colleagues observed that ventilation increased when the oxygen content of the blood flowing through the bifurcation of the common carotid artery was reduced (winning him the Nobel Prize in 1938), the search for the oxygen chemosensor responsible for the ventilatory response to hypoxia was largely considered accomplished.

The persistence of stimulatory effects of hypoxia in the absence (after surgical removal) of the carotid chemoreceptors (e.g. the carotid bodies) led other investigators, among them Julius Comroe, to ascribe hypoxic chemosensitivity to other sites, including both peripheral sites (e.g., aortic bodies) and central brain sites (e.g., hypothalamus, pons and rostral ventrolateral medulla). The aortic chemoreceptor, located in the aortic body, may also be an important chemoreceptor in humans with significant influence on vascular tone and cardiac function.

### Carotid Body Chemoreflex:

The carotid body is a small cluster of chemoreceptors (also known as glomus cells) and supporting cells located near, and in most cases directly at, the medial side of the bifurcation (fork) of the carotid artery, which runs along both sides of the throat.

These organs act as sensors detecting different chemical stimuli from arterial blood and triggering an action potential in the afferent fibers that communicate this information to the Central Nervous System (CNS). In response, the CNS activates reflexes that control heart rate (HR), renal function and peripheral blood circulation to maintain the desired homeostasis of blood gases, O₂ and CO₂, and blood pH. This closed loop control function that involves blood gas chemoreceptors is known as the carotid body chemoreflex (CBC). The carotid body chemoreflex is integrated in the CNS with the carotid sinus baroreflex (CSB) that maintains arterial blood pressure. In a healthy organism these two reflexes maintain blood pressure and blood gases within a narrow physiologic range. Chemosensors and barosensors in the aortic arch contribute redundancy and fine-tuning function to the closed loop chemoreflex and baroreflex.

The carotid sinus baroreflex is accomplished by negative feedback systems incorporating pressure sensors (e.g., baroreceptors) that sense the arterial pressure. Baroreceptors also exist in other places, such as the aorta and coronary arteries. Important arterial baroreceptors are located in the carotid sinus 202 [See FIGURE 1], a slight dilatation of the internal carotid artery 201 at its origin from the common carotid. The carotid sinus baroreceptors are close to but anatomically separate from the carotid body. Baroreceptors respond to stretching of the arterial wall and communicate blood pressure information to CNS. Baroreceptors are distributed in the arterial walls of the carotid sinus while the chemoreceptors (glomus cells) are clustered inside the carotid body. This makes the selective reduction of chemoreflex described in this application possible while substantially sparing the baroreflex.

The carotid body exhibits great sensitivity to hypoxia (low threshold and high gain). In chronic Congestive Heart Failure (CHF), the sympathetic nervous system activation that is directed to attenuate systemic hypoperfusion at the initial phases of CHF may ultimately exacerbate the progression of cardiac dysfunction that subsequently increases the extra-cardiac abnormalities, a positive feedback cycle of progressive deterioration, a vicious cycle with ominous consequences. It was thought that much of the increase in the sympathetic nerve activity (SNA) in CHF was based on an increase of sympathetic flow at a level of the CNS and on the depression of arterial baroreflex function. In the past several years, it has been demonstrated that an increase in the activity and sensitivity of peripheral chemoreceptors (heightened chemoreflex function) also plays an important role in the enhanced SNA that occurs in CHF.

### Role of Altered Chemoreflex in CHF:

As often happens in chronic disease states, chemoreflexes that are dedicated under normal conditions to maintaining homeostasis and correcting hypoxia contribute to increase the sympathetic tone in patients with CHF, even under normoxic conditions. The understanding of how abnormally enhanced sensitivity of the peripheral chemosensors, particularly the carotid body, contributes to the tonic elevation in SNA in patients with CHF has come from several studies in animals. According to one theory, the local angiotensin receptor system plays a fundamental role in the enhanced carotid body chemoreceptor sensitivity in CHF. In addition, evidence in both CHF patients and animal models of CHF has clearly established that the carotid body chemoreflex is often hypersensitive in CHF patients and contributes to the tonic elevation in sympathetic function. This derangement derives from altered function at the level of both the afferent and central pathways of the reflex arc. The mechanisms responsible for elevated afferent activity from the carotid body in CHF are not yet fully understood.

Regardless of the exact mechanism behind the carotid body hypersensitivity, the chronic sympathetic activation driven from the carotid body and other autonomic pathways leads to further deterioration of cardiac function in a positive feedback cycle. As CHF ensues, the increasing severity of cardiac dysfunction leads to progressive escalation of these alterations in carotid body chemoreflex function to further elevate sympathetic activity and cardiac deterioration. The trigger or causative factors that occur in the development of CHF that sets this cascade of events in motion and the time course over which they occur remain obscure. Ultimately, however, causative factors are tied to the cardiac pump failure and reduced cardiac output. According to one theory, within the carotid body, a progressive and chronic reduction in blood flow may be the key to initiating the maladaptive changes that occur in carotid body chemoreflex function in CHF.

There is sufficient evidence that there is increased peripheral and central chemoreflex sensitivity in heart failure, which is likely to be correlated with the severity of the disease. There is also some evidence that the central chemoreflex is modulated by the peripheral chemoreflex. According to current theories, the carotid body is the predominant contributor to the peripheral chemoreflex in humans; the aortic body having a minor contribution.

Although the mechanisms responsible for altered central chemoreflex sensitivity remain obscure, the enhanced peripheral chemoreflex sensitivity can be linked to a depression of nitric oxide production in the carotid body affecting afferent sensitivity, and an elevation of central angiotensin II affecting central integration of chemoreceptor input. The enhanced chemoreflex may be responsible, in part, for the enhanced ventilatory response to exercise, dyspnea, Cheyne-Stokes breathing, and sympathetic activation observed in chronic heart failure patients. The enhanced chemoreflex may be also responsible for hyperventilation and tachypnea (e.g. fast breathing) at rest and exercise, periodic breathing during exercise, rest and sleep, hypocapnia, vasoconstriction, reduced peripheral organ perfusion and hypertension.

### Dyspnea:

Shortness of breath, or dyspnea, is a feeling of difficult or labored breathing that is out of proportion to the patient's level of physical activity. It is a symptom of a variety of different diseases or disorders and may be either acute or chronic. Dyspnea is the most common complaint of patients with cardiopulmonary diseases.

Dyspnea is believed to result from complex interactions between neural signaling, the mechanics of breathing, and the related response of the central nervous system. A specific area has been identified in the mid-brain that may influence the perception of breathing difficulties.

The experience of dyspnea depends on its severity and underlying causes. The feeling itself results from a combination of impulses relayed to the brain from nerve endings in the lungs, rib cage, chest muscles, or diaphragm, combined with the perception and interpretation of the sensation by the patient. In some cases, the patient's sensation of breathlessness is intensified by anxiety about its cause. Patients describe dyspnea variously as unpleasant shortness of breath, a feeling of increased effort or tiredness in moving the chest muscles, a panicky feeling of being smothered, or a sense of tightness or cramping in the chest wall.

The four generally accepted categories of dyspnea are based on its causes: cardiac, pulmonary, mixed cardiac or pulmonary, and non-cardiac or non-pulmonary. The most common heart and lung diseases that produce dyspnea are asthma, pneumonia, COPD, and myocardial ischemia or heart attack (myocardial infarction). Foreign body inhalation, toxic damage to the airway, pulmonary embolism, congestive heart failure (CHF), anxiety with hyperventilation (panic disorder), anemia, and physical deconditioning because of sedentary lifestyle or obesity can produce dyspnea. In most cases, dyspnea occurs with exacerbation of the underlying disease. Dyspnea also can result from weakness or injury to the chest wall or chest muscles, decreased lung elasticity, obstruction of the airway, increased oxygen demand, or poor pumping action of the heart that results in increased pressure and fluid in the lungs, such as in CHF.

Acute dyspnea with sudden onset is a frequent cause of emergency room visits. Most cases of acute dyspnea involve pulmonary (lung and breathing) disorders, cardiovascular disease, or chest trauma. Sudden onset of dyspnea (acute dyspnea) is most typically associated with narrowing of the airways or airflow obstruction (bronchospasm), blockage of one of the arteries of the lung (pulmonary embolism), acute heart failure or myocardial infarction, pneumonia, or panic disorder.

Chronic dyspnea is different. Long-standing dyspnea (chronic dyspnea) is most often a manifestation of chronic or progressive diseases of the lung or heart, such as COPD, which includes chronic bronchitis and emphysema. The treatment of chronic dyspnea depends on the underlying disorder. Asthma can often be managed with a combination of medications to reduce airway spasms and removal of allergens from the patient's environment. COPD requires medication, lifestyle changes, and long-term physical rehabilitation. Anxiety disorders are usually treated with a combination of medication and psychotherapy.

Although the exact mechanism of dyspnea in different disease states is debated, there is no doubt that the CBC plays some role in most manifestations of this symptom. Dyspnea seems to occur most commonly when afferent input from peripheral receptors is enhanced or when cortical perception of respiratory work is excessive.

### Surgical Removal of the Glomus and Resection of Carotid Body Nerves:

A surgical treatment for asthma, removal of the carotid body or glomus (glomectomy), was described by Japanese surgeon Komei Nakayama in 1940s. According to Nakayama in his study of 4,000 patients with asthma, approximately 80% were cured or improved six months after surgery and 58% allegedly maintained good results after five years. Komei Nakayama performed most of his surgeries while at the Chiba University during World War II. Later in the 1950's, a U.S. surgeon, Dr. Overholt, performed the Nakayama operation on 160 U.S. patients. He felt it necessary to remove both carotid bodies in only three cases. He reported that some patients feel relief the instant when the carotid body is removed, or even earlier, when it is inactivated by an injection of procaine (Novocain).

Overholt, in his paper Glomectomy for Asthma published in Chest in 1961, described surgical glomectomy the following way: "A two-inch incision is placed in a crease line in the neck, one-third of the distance between the angle of the mandible and clavicle. The platysma muscle is divided and the sternocleidomastoid retracted laterally. The dissection is carried down to the carotid sheath exposing the bifurcation. The superior thyroid artery is ligated and divided near its take-off in order to facilitate rotation of the carotid bulb and expose the medial aspect of the bifurcation. The carotid body is about the size of a grain of rice and is hidden within the adventitia of the vessel and is of the same color. The perivascular adventitia is removed from one centimeter above to one centimeter below the bifurcation. This severs connections of the nerve plexus which surrounds the carotid body. The dissection of the adventitia is necessary in order to locate and identify the body. It is usually located exactly at the point of bifurcation on its medial aspect. Rarely, it may be found either in the center of the crotch or on the lateral wall. The small artery entering the carotid body is clamped, divided, and ligated. The upper stalk of tissue above the carotid body is then clamped, divided, and ligated."

In January, 1965, the New England Journal of Medicine published a report of 15 cases in which there had been unilateral removal of the cervical glomus (carotid body) for the treatment of bronchial asthma, with no objective beneficial effect. This effectively stopped the practice of glomectomy to treat asthma in the U.S.

Winter developed a technique for separating nerves that contribute to the carotid sinus nerves into two bundles, carotid sinus (baroreflex) and carotid body (chemoreflex), and selectively cutting out the latter. The Winter technique is based on his discovery that carotid sinus (baroreflex) nerves are predominantly on the lateral side of the carotid bifurcation and carotid body (chemoreflex) nerves are predominantly on the medial side.

### Neuromodulation of the Carotid Body Chemoreflex:

Hlavaka in U.S. Patent Application Publication 2010/0070004 filed August 7, 2009, describes implanting an electrical stimulator to apply electrical signals, which block or inhibit chemoreceptor signals in a patient suffering dyspnea. Hlavaka teaches that "some patients may benefit from the ability to reactivate or modulate chemoreceptor functioning." Hlavaka focuses on neuromodulation of the chemoreflex by selectively blocking conduction of nerves that connect the carotid body to the CNS. Hlavaka describes a traditional approach of neuromodulation with an implantable electric pulse generator that does not modify or alter tissue of the carotid body or chemoreceptors.

The central chemoreceptors are located in the brain and are difficult to access. The peripheral chemoreflex is modulated primarily by carotid bodies that are more accessible. Previous clinical practice had very limited clinical success with the surgical removal of carotid bodies to treat asthma in 1940s and 1960s.

### Overview:

Ablation of a peripheral chemoreceptor (e.g. carotid body or aortic body) in patients having sympathetically mediated disease and high peripheral chemosensitivity has been conceived to reduce peripheral chemosensitivity and consequentially reduce afferent signaling from peripheral chemoreceptors to the central nervous system. The expected reduction of chemoreflex sensitivity to hypoxia and other stimuli such as blood flow, blood CO₂, glucose concentration or blood pH can directly reduce afferent signals from chemoreceptors and produce at least one beneficial effect such as the reduction of central sympathetic activation, reduction of the sensation of breathlessness (dyspnea), vasodilation, increase of exercise capacity, reduction of blood pressure, reduction of sodium and water retention, reduction of insulin resistance, reduction of hyperventilation, reduction of tachypnea, reduction of hypocapnia, or improve symptoms of a sympathetically mediated disease and may ultimately slow down the disease progression and extend life. It is understood that a sympathetically mediated disease that may be treated with carotid body ablation may comprise elevated sympathetic tone, an elevated sympathetic/parasympathetic activity ratio, autonomic imbalance primarily attributable to central sympathetic tone being abnormally high, or heightened sympathetic tone at least partially attributable to afferent excitation traceable to hypersensitivity or hyperactivity of a peripheral chemoreceptor (e.g. carotid body). In some important clinical cases where baseline hypocapnea is present, reduction of hyperventilation may be expected. It is understood that hyperventilation in the context of this invention means respiration in excess of metabolic needs on the individual that generally leads to slight but significant hypocapnea (blood CO₂ partial pressure below normal of approximately 40 mmHg, for example in the range of 33 to 38 mmHg) .

Patients having CHF or hypertension concurrent with heightened peripheral chemoreflex sensitivity often react as if their system was hypercapnic even if it is not. The reaction is to hyperventilate, a maladaptive attempt to rid the system of CO₂, thus overcompensating and creating a hypocapnic and alkalotic system. Some researchers attribute this hypersensitivity / hyperactivity of the carotid body to the direct effect of catecholamines, hormones circulating in excessive quantities in the blood stream of CHF patients. The present invention may be particularly useful to treat such patients who are hypocapnic and possibly alkalotic resulting from high tonic output from carotid bodies. Such patients are particularly predisposed to periodic breathing and central apnea hypopnea type events that cause arousal, disrupt sleep, cause intermittent hypoxia and are by themselves detrimental and difficult to treat.

It is appreciated that periodic breathing of Cheyne Stokes pattern occurs in patients during sleep, exercise and even at rest as a combination of central hypersensitivity to CO₂, peripheral chemosensitivity to O₂ and CO₂ and prolonged circulatory delay. All these parameters are often present in CHF patients that are at high risk of death. Thus, patients with hypocapnea, CHF, high chemosensitivity and prolonged circulatory delay, and specifically ones that exhibit periodic breathing at rest or during exercise or induced by hypoxia are likely beneficiaries of the proposed therapy.

Hyperventilation is defined as breathing in excess of a person's metabolic need at a given time and level of activity. Hyperventilation is more specifically defined as minute ventilation in excess of that needed to remove CO₂ from blood in order to maintain blood CO₂ in the normal range (e.g. around 40 mmHg partial pressure). For example, patients with arterial blood PCO₂ in the range of 32-37 mmHg can be considered hypocapnic and in hyperventilation.

For the purpose of this disclosure hyperventilation is equivalent to abnormally low levels of carbon dioxide in the blood (e.g. hypocapnia, hypocapnea, or hypocarbia) caused by overbreathing. Hyperventilation is the opposite of hypoventilation (e.g. underventilation) that often occurs in patients with lung disease and results in high levels of carbon dioxide in the blood (e.g. hypercapnia or hypercarbia).

A low partial pressure of carbon dioxide in the blood causes alkalosis, because CO₂ is acidic in solution and reduced CO₂ makes blood pH more basic, leading to lowered plasma calcium ions and nerve and muscle excitability. This condition is undesirable in cardiac patients since it can increase probability of cardiac arrhythmias.

Alkalemia may be defined as abnormal alkalinity, or increased pH of the blood. Respiratory alkalosis is a state due to excess loss of carbon dioxide from the body, usually as a result of hyperventilation. Compensated alkalosis is a form in which compensatory mechanisms have returned the pH toward normal. For example, compensation can be achieved by increased excretion of bicarbonate by the kidneys.

Compensated alkalosis at rest can become uncompensated during exercise or as a result of other changes of metabolic balance. Thus the invented method is applicable to treatment of both uncompensated and compensated respiratory alkalosis.

Tachypnea means rapid breathing. For the purpose of this disclosure a breathing rate of about 6 to 16 breaths per minute at rest is considered normal but there is a known benefit to lower rate of breathing in cardiac patients. Reduction of tachypnea can be expected to reduce respiratory dead space, increase breathing efficiency, and increase parasympathetic tone.

### Targets:

To inhibit or suppress a peripheral chemoreflex, anatomical targets for ablation (e.g. targeted tissue or target sites) may include at least one carotid body, an aortic body, nerves associated with a peripheral chemoreceptor, small blood vessels feeding a peripheral chemoreceptor, carotid body parenchyma, chemosensitive cells (e.g. glomus cells), or a combination thereof.

An ablation may be focused exclusively on targeted tissue, or be focused on the targeted tissue while safely ablating tissue proximate to the targeted tissue (e.g. to ensure the targeted tissue is ablated or as an approach to gain access to the targeted tissue). An ablation may be as big as the peripheral chemoreceptor (e.g. carotid body or aortic body) itself, somewhat smaller, or bigger and can include tissue surrounding the chemoreceptor such as blood vessels, adventitia, fascia, small blood vessels perfusing the chemoreceptor, or nerves connected to and innervating the glomus cells.

Tissue may be ablated to inhibit or suppress a chemoreflex of only one of a patient's two carotid bodies. Another embodiment involves ablating tissue to inhibit or suppress a chemoreflex of both of a patient's carotid bodies. For example a therapeutic method may include sequential ablation of one carotid body, measurement of resulting chemosensitivity and ablation of the second carotid body if needed to further reduce chemosensitivity following unilateral ablation.

An embodiment of a therapy of the present invention may substantially reduce chemoreflex without substantially reducing the baroreflex of the patient. The proposed ablation procedure may be targeted to substantially spare the carotid sinus, baroreceptors distributed in the walls of carotid arteries (specifically internal carotid artery), and at least some of the carotid sinus nerves that conduct signals from said baroreceptors. For example, the baroreflex may be substantially spared by targeting a limited volume of ablated tissue possibly enclosing the carotid body, tissues containing a substantial number of carotid body nerves, tissues located in adventitia of a medial segment of a carotid bifurcation, tissue located at the attachment of a carotid body to an artery, or extending to tissues located on the medial side of a carotid artery bifurcation saddle and avoiding damage to the lateral side. Said targeted ablation is enabled by visualization of the area or carotid body itself, for example by CT, ultrasound sonography, fluoroscopy, blood flow visualization, or injection of contrast, and positioning of an instrument in the carotid body or in close proximity while avoiding excessive damage (e.g. perforation, stenosis, thrombosis) to carotid arteries, baroreceptors or carotid sinus nerves. Thus imaging a carotid body before ablation may be instrumental in (a) selecting candidates if CB is present, large enough and identified and (b) guiding therapy by providing a landmark map for an operator to guide an ablation instrument to carotid body nerves, the area of a blood vessel proximate to a carotid body, or to an area where carotid body nerves may be anticipated.

Once a carotid body is removed or denervated, the carotid body chemoreflex does not substantially return in humans (in humans aortic chemoreceptors are considered undeveloped). To the contrary, once a carotid sinus baroreflex is removed it is generally compensated, after weeks or months, by the aortic baroreceptor baroreflex. Thus, if both the carotid chemoreflex and baroreflex are removed, for example by interruption of the carotid sinus nerve, baroreflex may eventually be restored while the chemoreflex may not. The consequences of temporary removal of the baroreflex can be relatively severe and require hospitalization and management with drugs, but they generally are not terminal or permanent. Thus, it is understood that while selective (with baroreflex preservation) removal of carotid body chemoreflex is desired, it may not be absolutely necessary in some cases.

### Thermal Ablation:

Embodiments of the present invention may include systems for the transvascular thermal ablation of tissue for the complete or partial ablation of a carotid body via thermal heating or cooling mechanisms to achieve a reduction of carotid body chemoreflex. Several methods disclosed herein form lesions at, or proximate to, the carotid body, which permanently (or for at least an extended period) suppresses or inhibits natural chemoreceptor functioning, which is in contrast to neuromodulating or reversibly deactivating and reactivating chemoreceptor functioning.

Thermally-induced ablation may be achieved via an apparatus positioned proximate to targeted tissue that may include chemoreceptor cells, afferent nerves, or nerve endings (e.g. neural fibers). The apparatus may be, for example, positioned within a carotid artery vasculature (e.g., positioned intravascularly for example in an external carotid artery), positioned extravascularly, positioned intra-to-extravascularly, positioned percutaneously, positioned surgically via incision, or a combination thereof. Thermal destruction of tissue (e.g. thermal ablation) can be achieved by either heating or cooling (e.g. cryo-ablation) and may be due to a direct effect on tissues and structures that are induced by the thermal stress. Additionally or alternatively, the thermal disruption may at least in part be due to alteration of vascular or peri-vascular structures (e.g. arteries, arterioles, capillaries or veins), which perfuse the carotid body and neural fibers surrounding the carotid body (e.g. nerves that transmit afferent information from carotid body chemoreceptors to the brain). Additionally or alternatively thermal disruption may be due to a healing process, fibrosis, or scarring of tissue following thermal injury, particularly when prevention of regrowth and regeneration of active tissue is desired. Common to these thermally-induced ablation procedures the afferent neural signaling from the carotid body is reduced or removed and the chemoreflex sensitivity is reduced, as is generally indicated by a reduction of an increase of ventilation and ventilation effort per unit of blood gas change and by a reduction of central sympathetic nerve activity that can be measured indirectly. Sympathetic nerve activity can be assessed by measuring activity of peripheral nerves leading to muscles (MSNA), heart rate (HR), heart rate variability (HRV), production of hormones such as renin, epinephrine and angiotensin, and peripheral vascular resistance. All these parameters are measurable and can lead directly to the health improvements. In the case of CHF patients blood pH, blood PCO₂, degree of hyperventilation and metabolic exercise test parameters such as peak VO₂, and VE/VCO₂ slope are equally important. It is believed that patients with heightened chemoreflex have low VO₂ and high VE/VCO₂ slope (index of respiratory efficiency) as a result of tachypnea and low blood CO₂. These parameters are also firmly related exercise limitations that further speed up patient's status deterioration towards morbidity and death.

Thermal disruption includes inducement of any mechanism that results in an inability or reduction of ability of the carotid body to transduce or transmit information to the brain (specifically to the brain medulla via the nerve of Hering) or other organs/sensors that result in the negative physiological and clinical events previous noted. This reduction of carotid body ability to transmit information to the brain may be the reduction in tonic nerve activity or response to acute hypoxia or hypoxemia. It is accepted that the carotid body responds primarily to hypoxia but also responds to carbon dioxide, hydrogen ion, blood pH and glucose concentration. It can also manifest as a reduction of response to intermittent, for example nocturnal, hypoxia.

Nerve of Hering is a branch of the cranial nerve IX (glossopharyngeal nerve). The glossopharyngeal nerve synapses in the nucleus tractus solitarius (NTS) located in the medulla of the brainstem. Anatomically the nerve of Hering is a branch of the glossopharyngeal nerve to the carotid sinus and the carotid body. It is the nerve that runs downwards anterior to the internal carotid artery and communicates with the vagus and sympathetic chain and then divides in the angle of bifurcation of the common carotid artery to supply the carotid body and carotid sinus. It carries impulses from the baroreceptors in the carotid sinus, to help maintain a more consistent blood pressure, and from chemoreceptors in the carotid body via separate nerve fibers. It is also known as "Hering's nerve".

As used herein, thermal heating mechanisms for ablation include both thermal ablation and non-ablative thermal injury or damage (e.g., via sustained heating or resistive heating). Thermal heating mechanisms may include raising the temperature of target neural fibers above a desired threshold, for example, above a body temperature of about 37°C e.g., to achieve non-ablative thermal injury, or above a temperature of about 45°C (e.g. above about 60°C) to achieve ablative thermal injury.

As used herein, thermal-cooling mechanisms for ablation may include reducing the temperature of target neural fibers below a desired threshold (e.g. to achieve freezing thermal injury). It is generally accepted that temperatures below -40°C applied over a minute or two results in irreversible necrosis of tissue and scar formation.

In addition to monitoring or controlling the temperature during thermal ablation, a length of exposure to thermal stimuli may be specified to affect an extent or degree of efficacy of the thermal ablation. For example, the length of exposure to thermal stimuli may be longer than instantaneous exposure (e.g. longer than about 30 seconds, or even longer than 2 minutes). Furthermore, the length of exposure can be less than 10 minutes, though this should not be construed as the upper limit of the exposure period.

When conducting ablation via thermal mechanisms, the temperature threshold discussed previously may be determined as a function of the duration of exposure to thermal stimuli. Additionally or alternatively, the length of exposure may be determined as a function of the desired temperature threshold. These and other parameters may be specified or calculated to achieve and control desired thermal ablation.

In some embodiments, thermally-induced ablation of glomus cells may be achieved via direct application of thermal cooling or heating energy to the target neural fibers. For example, a chilled or heated fluid can be applied at least proximate to the target, or heated or cooled elements (e.g., thermoelectric element, resistive heating element, cryogenic tip or balloon) can be placed in the vicinity of the glomus. In other embodiments, thermally-induced ablation may be achieved via indirect generation or application of thermal energy to the target neural fibers, such as through application of an electric field (e.g. radiofrequency, alternating current, and direct current), high-intensity focused ultrasound (HIFU), laser irradiation, or microwave radiation, to the target neural fibers. For example, thermally induced ablation may be achieved via delivery of a pulsed or continuous thermal electric field to the target tissue, the electric field being of sufficient magnitude or duration to thermally induce ablation of the target tissue (e.g., to heat or thermally ablate or cause necrosis of the glomus). Additional and alternative methods and apparatuses may be utilized to achieve thermally induced ablation, as described hereinafter.

### Surgical Approach:

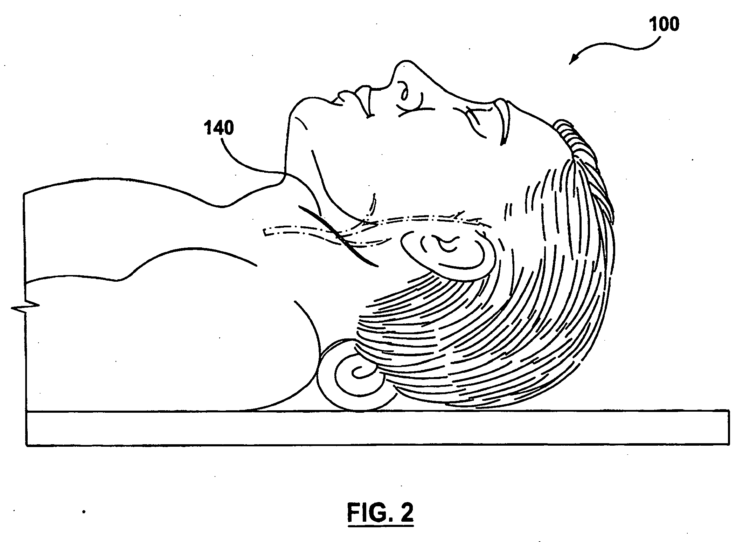

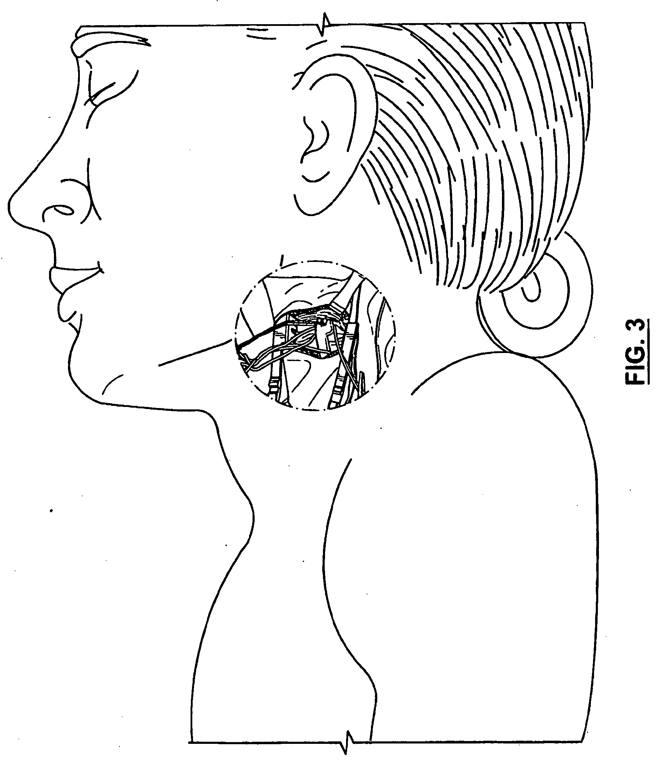

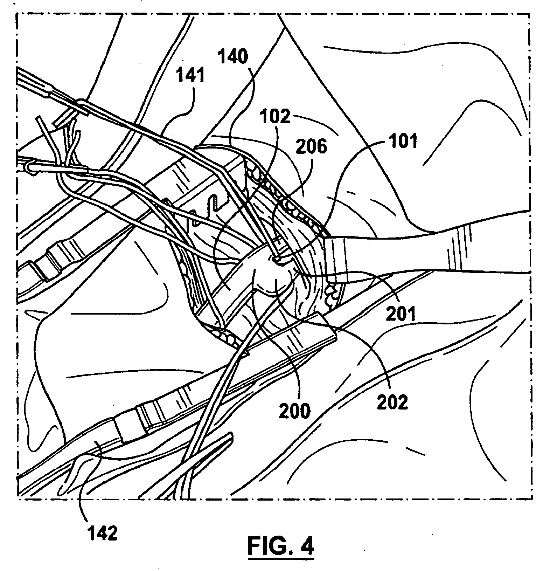

An embodiment of the present invention comprises a surgical technique (e.g. open surgery, laparoscopic, endoscopic, robotically assisted) to gain access to a carotid body or carotid body area. For example, a surgical approach may comprise steps known in the art for surgically accessing a carotid body for glomectomy or tumor removal. As shown in FIGURES 2 to 4, a carotid bifurcation 200 can be exposed through an incision 140 in a patient's skin revealing a common carotid artery 102, an internal carotid artery 201, a carotid sinus 202, an external carotid artery 206, and a carotid body 101. The structures may be identified and restrained with string or clamps to visually expose and stabilize a carotid body 101.

Optionally, prior to obtaining surgical access to a carotid body, non-invasive imaging (e.g. CT, MRI, sonography) of a patient's carotid area may be performed to assess the location and morphology of the carotid body and surrounding structures. The imaging may be used to ascertain risk assessment of surgical access and ablation. It may be used in combination with other patient assessment results (e.g. chemosensitivity) to guide a decision to proceed with a surgical carotid body ablation procedure or not. The imaging may further be used to guide a surgical procedure. For example, an understanding of relative position and morphology of anatomical structures as seen in images may assist a surgeon to obtain surgical access to a carotid body. Furthermore, if a carotid body is difficult to find or is not visible during surgery, images may be used to determine a most probable location of a target site and reduce unnecessary discretion.

Optionally, when the carotid bifurcation is visually exposed a stimulation electrode may be placed in contact with a structure suspected to be a carotid body. The stimulation electrode may be located on the distal tip of a stimulation probe. Alternatively, the stimulation electrode may be located on an ablation device such as a radiofrequency electrode. A stimulation electrode may be an electrically conductive surface (e.g. stainless steel) that delivers stimulation current to tissue in contact. The stimulation electrode may be electrically connected with a wire to a stimulation signal generator. A return electrode (e.g. dispersive electrode patch) may be placed on the patient's skin to complete the electrical circuit. Optionally, the stimulation signal generator may communicate with physiological monitors (e.g. equipment that monitors heart rate, ventilation, blood pressure, blood flow) so a correlation may be made between stimulation and physiological effect. Electrical stimulation may be used to confirm contact with or sufficient proximity to a carotid body and non-contact with or sufficient distance from a baroreceptor prior to ablation, as will be discussed in more detail later.

The carotid body can be ablated using a surgical tool. In one embodiment of a surgical technique a carotid body is tied off tightly and cut off using a scalpel or a harmonic scalpel. An RF heated snare can be also used to reduce bleeding. In another embodiment a carotid body is crushed using a tool such as clamp forceps or cryogenic forceps. In another embodiment a carotid body is ablated using electrocautery forceps. A benefit of electrocautery is control of bleeding and barrier to potential re-innervation and re-growth of the carotid body chemosensitive cells. In another embodiment a carotid body is ablated with RF. An RF electrode on the tip of a probe may be held in contact with, or inserted into the carotid body (or tissue where a carotid body is expected to reside if it is not entirely visible and obscured by fibrous or nerve tissues) while RF energy is applied. In another embodiment a carotid body is ablated using a cryogenic probe. In another embodiment a carotid body may be ablated by applying a cryogen such as liquid nitrogen directly to the carotid body.

Optionally, if there is any doubt that the carotid body was ablated, electrical, mechanical, or chemical stimulation may be used during or following the ablation procedure to confirm ablation. To confirm that baroreflex is intact mechanical, electrical, chemical stimulation of the carotid sinus can be used during the procedure.

### Endovascular Catheter:

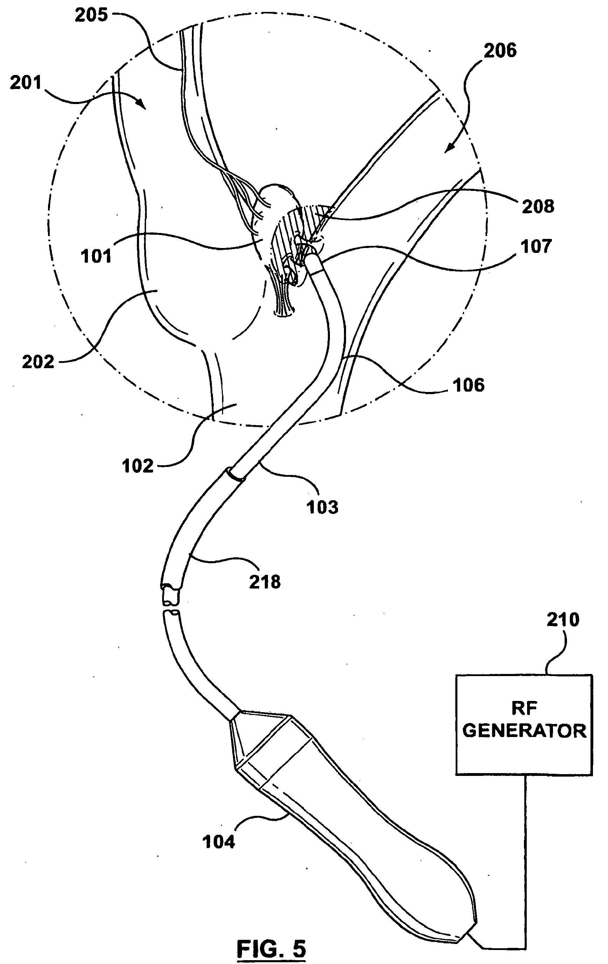

An embodiment of the present invention is shown in FIGURE 5. A patient who is suffering from a cardiac, metabolic or pulmonary disease involving heightened SNS (e.g. dyspnea, hypertension, COPD or CHF), may be treated with a carotid body ablation catheter 103. The carotid body ablation catheter 103 may be inserted through a patient's vasculature to a common carotid artery 102 and applied to ablate a carotid body 101 associated with the common carotid artery 102. A carotid body ablation catheter 103 may ablate a carotid body or associated tissues (e.g. nerve supply, blood supply) through transvascular access (e.g. accessing a target across the wall of a blood vessel). For example, ablative energy may be applied to or through a blood vessel wall to reach the target tissue, or an element in association with a carotid body ablation catheter may puncture through a vessel wall to reach the target tissue. Transvascular access may be applied from an external carotid artery 206, an internal carotid artery 201, a common carotid artery 102, a carotid bifurcation 202, or a combination thereof. The carotid body ablation catheter 103 may comprise an energy delivery element 107 located at the distal tip. The energy delivery element 107 at the distal tip of the catheter 103 is shown in contact with the EC 206 vessel wall proximate to carotid body 101 in the process of creating a lesion 208. Optionally, multiple small lesions may be created to reduce trauma from a single larger lesion. For example, carotid body location can be identified using a selected imaging modality (e.g. CT, sonography, MRI, fluoroscopy) and the endovascular area proximate to the carotid body may be treated with multiple ablations. Tissue contact is generally needed for energy delivery that is dependent on thermal or electrical conduction (e.g. radiofrequency, thermal conduction, and cryogenic ablation) but may be less necessary for energies such as focused ultrasound or microwave ablation. Different modalities of delivering thermal energy to ablate tissue have distinct advantages and disadvantages. The proximal end of the catheter 103 may have a handle 104, which remains outside of the body. The energy delivery element 107 of the carotid body ablation device 103 applies energy to the carotid body to, for example, ablate the carotid body. Alternatively, a carotid body ablation catheter 103 may deliver a substance to ablate the carotid body, such as an ablative chemical or deliver material to embolize the carotid body.

An endovascular catheter may be delivered into a patient's vasculature via common approaches including femoral, radial, brachial artery or vein access, or even via a cervical approach directly into a carotid artery. An endovascular procedure may involve the use of a guidewire, delivery sheath, guide catheter, or introducer.

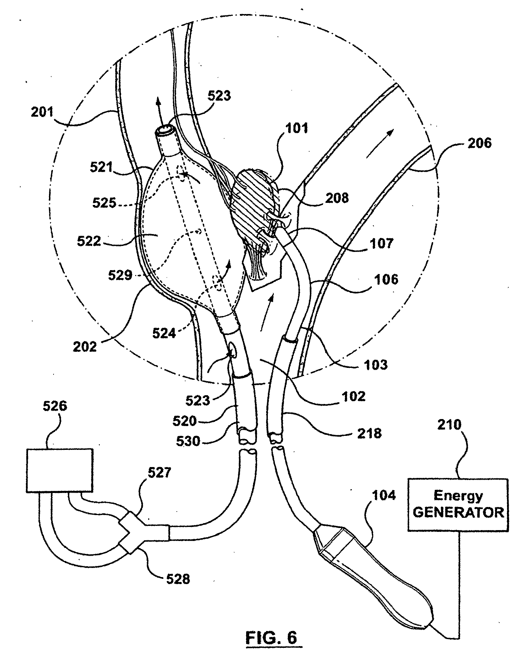

There may be danger of creating a brain embolism while performing an endovascular procedure in a patient's carotid artery, for example, a thrombus may be created by delivering ablation energy such as on a radiofrequency electrode, a piece of atheromatous plaque may be dislodged by catheter movement, or a cryogenic catheter could release a piece of frozen blood. In one embodiment of the present invention, in addition to a carotid body ablation catheter, an endovascular catheter is used to place a brain embolism protection device in a patient's internal carotid artery during a carotid body ablation procedure. An embodiment of this invention comprises occluding a patient's internal carotid artery. Blood flowing from a common carotid artery 102 would not flow through a connecting internal carotid artery 201, which feeds the brain, but instead would flow through the external carotid artery, which feeds other structures of the head that are much more capable of safely receiving an embolism. For example, as shown in FIGURE 6 a brain embolism protection device in the form of an inflatable balloon 521 is placed in an internal carotid artery 201. An expandable device such as this may also facilitate endovascular ablation of a carotid body from an external carotid artery by pushing the carotid body closer to the wall of the external carotid artery and thus a lesion formed through the vessel wall of the external carotid artery may ablate the carotid body more effectively. The balloon 521 may be made from a soft, stretchable, compliant balloon material such as silicone and may be inflated with a fluid (e.g. saline or contrast agent) through an inflation lumen 524. Inflation fluid may be injected into an inlet port 527 by a syringe or by a computer controlled pump system 526. The balloon 521 may be placed, using a delivery sheath 530, at an,ostium of the internal carotid artery, in an internal carotid artery a short distance (e.g. up to about 3cm) from its ostium, or in a carotid sinus as shown in FIGURE 6.

Contrast solution may be injected into the common carotid artery 102, for example through the delivery sheath 530 to allow radiographic visualization of the common 102, internal 201 and external 206 carotid arteries, which may assist a physician to position the occlusion balloon 521 or confirm occlusion. An endovascular ablation catheter 103 may place an energy delivery element 107 proximate a carotid body, for example at an inner wall of a medial segment of a carotid bifurcation or of an external carotid artery. It is expected that blood flow would carry any debris into the external carotid artery where it is harmless. Occlusion of an internal carotid artery may be done for a period of time that allows an ablation procedure and that is safe for the brain (e.g. less than or equal to about 3 minutes, or between about 1 to 2 minutes). Optionally, an occlusion catheter 520 may comprise a blood flow lumen 523 providing fluid communication from a vessel space proximal to an occluding balloon 521 to a vessel space distal to the occluding balloon 521. The proximal opening of lumen 523 may be spaced a sufficient distance from the occluding balloon such that blood entering the lumen is upstream from an ablation and clean of debris that could be caused by the ablation. The blood flow lumen 523 could optionally be used to deliver an occlusion catheter 520 over a guide wire (not shown), such as a rapid exchange guide wire or conventional guide wire. After the carotid body is ablated the brain embolism protection device may be deployed and removed from the patient or positioned on the patient's contralateral side in the event of ablating the contralateral carotid body.

When used in conjunction with a carotid body ablation catheter 103 that delivers a heat generating energy such as radiofrequency energy, microwave or ultrasound, an occlusive brain embolism protection device, as shown in FIGURE 6 may provide additional benefit of increasing blood flow over an energy delivery element 107 and the vessel wall in contact with it. Increased blood flow may increase convective removal of heat from the energy delivery element and vessel wall allowing more energy to be delivered (e.g. greater power) to create a deeper ablation without adversely overheating the tissue or energy delivery element and avoiding unwanted tissue desiccation, tissue impedance rise, or thrombus formation.

It may be desirable to preserve a patent's baroreceptor located at the patient's internal carotid artery during a carotid body ablation procedure. An occlusion balloon may optionally be configured to cool surrounding tissue and provide thermal protection to the surrounding tissue during a thermal ablation procedure (e.g. RF, microwave, ultrasound ablation). As shown in FIGURE 6, balloon 521 is inflated with a circulating fluid. Alternatively, an occluding balloon may be cooled by a non-circulating chilled fluid or be cooled by an endothermic phase change of a fluid such as Nitrous Oxide N₂O. Tissue surrounding the thermal protection, occluding balloon may be cooled to a temperature insufficient to cause cryogenic injury but sufficient to maintain a non-ablative temperature when subjected to ablative energy of a carotid body ablation catheter. For example, tissue temperature may be maintained at a temperature in a range below about 42°C and above about -20°C (e.g. in a range of about 5°C to 37°C or in a range of about 25°C to 35°C).

In another embodiment a brain embolism protection device may be a blood-permeable filter deployed in a patient's internal carotid artery. A filter may be a fine mesh or net connected to a deployable frame that expands to envelop a cross-section of an internal carotid artery distal to a bifurcation. Other embodiments of a blood-permeable filter may include wire-type expandable devices such as baskets or umbrellas. Such a filter may allow antegrade blood flow to continue to the brain while trapping and retrieving debris in the blood, preventing a brain embolism. Such a device may be deployed in an internal carotid artery prior to the placement of ablation catheter and retrieved following ablation.

An energy field generator 210 may be located external to the patient. Various types of energy generators or supplies, such as electrical frequency generators, ultrasonic generators, and heating or cryogenic fluid supplies, may be used to provide energy to the energy delivery element at the distal tip of the catheter. An electrode or other energy applicator at the distal tip of the catheter should conform to the type of energy generator coupled to the catheter. The generator may include computer controls to automatically or manually adjust frequency and strength of the energy applied to the catheter, timing and period during which energy is applied, and safety limits to the application of energy. It should be understood that embodiments of energy delivery electrodes described hereinafter may be electrically connected to the generator even though the generator is not explicitly shown or described with each embodiment.

An ablated tissue lesion at or near the carotid body may be created by the application of thermal energy from the energy delivery element 107 proximate to the distal end of the carotid body ablation device 103. The ablated tissue lesion may disable the carotid body 101 or may suppress the activity of the carotid body. The disabling or suppression of the carotid body reduces the responsiveness of the glomus cells to changes of blood gas composition and effectively reduces the chemoreflex gain of the patient 100.

### RF catheter:

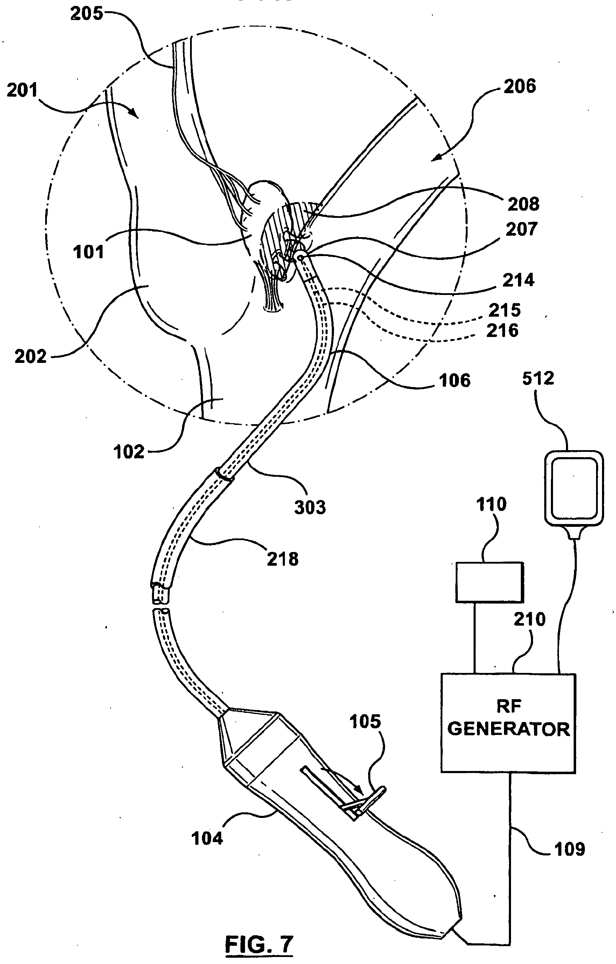

A carotid body ablation catheter may be a configured to deliver radiofrequency electrical current (RF) and as such, the energy delivery element 107 may be a radiofrequency electrode 207, as shown in FIGURE 7. RF is a rapidly alternating current that ablates tissue by generating heat in the tissue through ionic agitation, which is typically proportional to current density. Other factors that influence temperature generated in tissue include heat sinks (e.g. thermal convection due to blood flow) and tissue impedance. The volume of heated tissue is dependent on factors such as electrode size, RF power, duration of RF delivery, and waveform characteristics such as pulsing. In the embodiment shown in FIGURE 7, the carotid body ablation catheter 303 is connected by wires 109 to an RF energy generator 210. The generator 210 may be included with a computer controller 110 that controls the application of energy to the electrode 207. A reference electrode 212 is placed on the surface of the body of the patient 100 such as on the skin of the chest or the back of the patient. The reference electrode 212 establishes a current return path to the RF generator 210 for current flowing from the electrode 207, through the body of the patient and to the reference electrode 212. The arrangement in which current flows through a reference electrode 212 and an active electrode 207 is generally referred to as a monopolar arrangement. The reference electrode 212 has a relatively large surface area to minimize current density and avoid skin burns.

A RF electrode 207 may be made from an electrically conductive material (e.g. stainless steel, gold, platinum-iridium) and may be less than or equal to about 3 mm in diameter (e.g. between 1 to 2.5 mm) and less than or equal to about 5 mm in length (e.g. between 1 and 3mm). A temperature sensor 214 (e.g. thermocouple, thermistor, fluoroptic thermometry sensor) may be located in, near, or at the surface of the RF electrode 207. In FIGURE 7 a thermocouple 214 is located in the RF electrode 207 and is connected to two conductors 215 and 216. The conductors travel through the catheter body (e.g. through a lumen) from the distal to proximal end and are connected to wires 109 allowing the thermocouple to communicate with the RF generator 210. Conductors 215 and 216 may be, for example a copper and constantan conductor, respectively, such that joining the conductors 215 and 216 via solder, laser welding or the like creates a thermocouple junction. Copper conductor 215 may be used to carry both a thermocouple signal and deliver RF energy to the electrode 207 as shown in FIGURE 7. Alternatively, a separate conductor (not shown) may deliver RF energy to the electrode. The catheter 303 may further comprise deflectable section 106 near the distal end, for example within about 3cm from the distal end of the catheter 303. Deflectable section 106 may be controllably deflected by a physician by initiating an actuator 105 integrated in to a handle 104. For example, the actuator may be connected to a control wire (not shown) that travels the length of the catheter (e.g. through a lumen) to the deflectable section 106. The control wire may be connected to a structure that is biased to compression or tension of the control wire such that the structure deforms in its biased configuration. Other alternative embodiments for controlling deformation of the deflectable section may be used for example, electrically or thermally activating a shape memory alloy structure, or electrically activating an electroactive polymer structure.

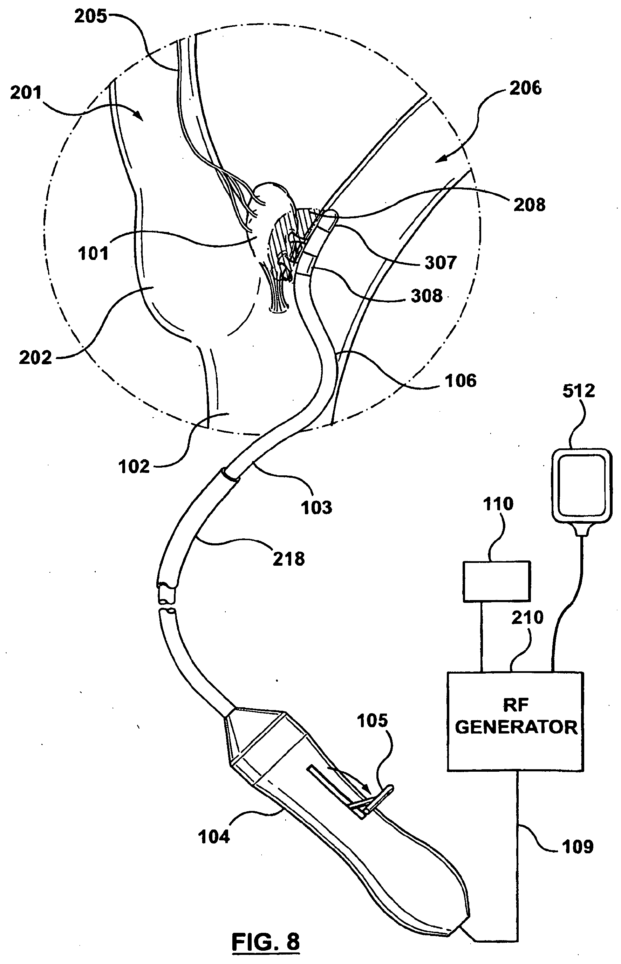

Alternatively, as shown in FIGURE 8, multiple electrodes may be arranged at or near the distal tip region of a carotid body ablation catheter 103 such that current flows from an active electrode 307 to a return electrode 308 to create an energy field, (e.g. an electric field) in the region adjacent the electrodes 307 and 308 that ablates tissue. Such an arrangement is generally referred to as a bipolar configuration. Active and return electrodes may be located on the same shaft of a catheter as shown in FIGURE 8. For example, the electrodes may be about the same size and shape and be distanced between about 0.5 mm and 10 mm (e.g. between about 1 and 4 mm) apart from one another. Alternatively, the electrodes may be different sizes so current density is greater around the smaller electrode creating a greater thermal effect. Another embodiment of a bipolar arrangement involves having an active electrode and a return electrode placed on separate shafts (not shown). Bipolar and monopolar electrosurgery techniques are well known in the field of catheters for RF ablation of tissue.

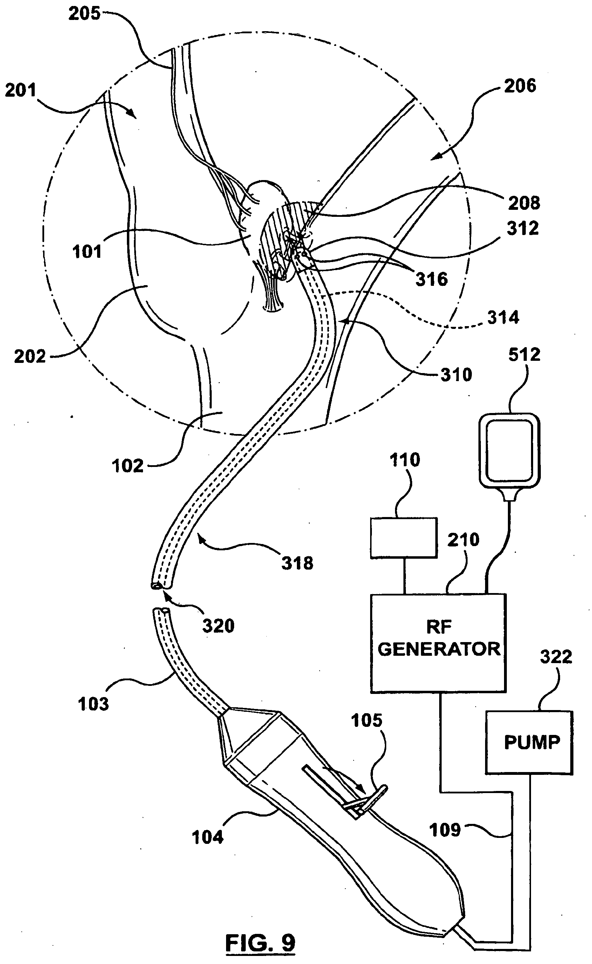

As shown in FIGURE 9, a RF ablation catheter may additionally be configured to provide cooled RF energy delivery. For example, a catheter 318 may contain a lumen 314 in fluid communication with one or more RF electrodes 312 to irrigate a cooling fluid 320 (e.g. room temperature or chilled saline) to the RF electrode. The cooling fluid may exit the RF electrode through irrigation ports 316 and enter the blood stream. Alternatively, cooling fluid may be circulated through a cavity or lumen in a cooled RF electrode and then circulate back through a lumen in the catheter shaft to be deposited elsewhere in the patient's vasculature or outside the body. A cooled RF system may additionally comprise a cooling fluid source and pump 322. The benefit of cooling a RF electrode may be reduction of the risk of heating blood, which may create a clot or emboli. Furthermore, cooled RF may produce ablations deeper in the tissue or may heat the surface layers of the tissue less. These benefits may be particularly advantageous in a carotid vasculature since the internal carotid feeds the brain and the targeted tissue may be beyond the vessel's surface (e.g. 2 to 5mm deep from the inner surface of a vessel wall).

### Cryogenic Ablation Catheter:

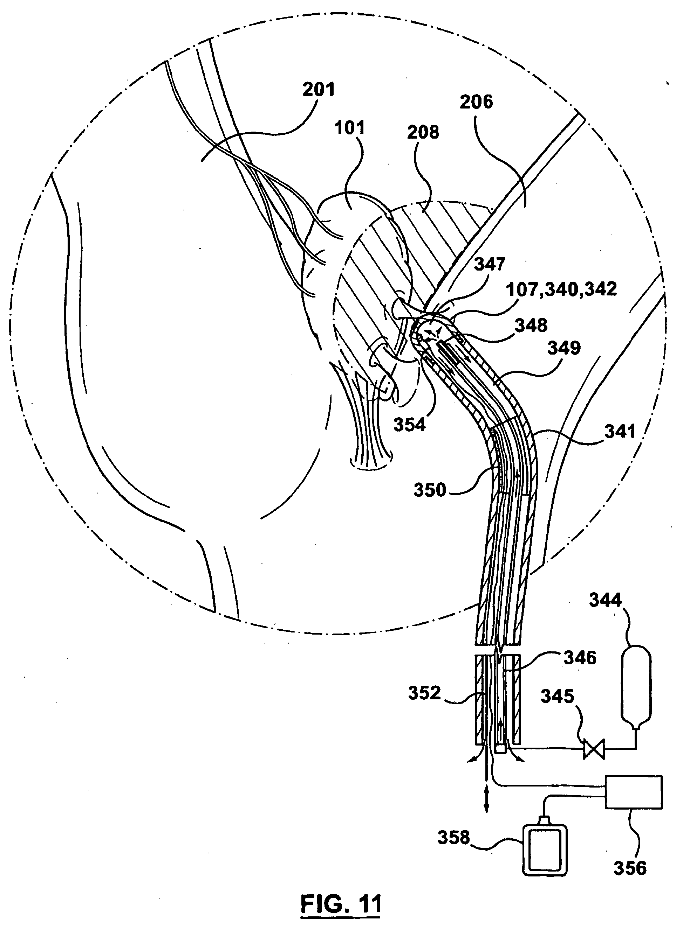

A carotid body ablation catheter may be configured to cryogenically ablate tissue and as such, the energy delivery element 107 may be a cryogenic applicator 340, as shown in FIGURE 11. Cryogenic ablation may possess benefits for a carotid body ablation procedure. For example, a non-ablative cold temperature (e.g. - 15 to 5°C) may be induced in a carotid body to temporarily block activity, which may be used to test if a permanent carotid body ablation would have desirable effects such as decreasing chemosensitivity. Other benefits may include less pain or less risk of vessel stenosis compared to high temperature ablation, a deep ablation, and cryo-adhesion, wherein a cryogenic applicator may stick to a vessel wall when cold, which could provide excellent contact stability. Another benefit is that the formation of ice in tissue may be viewed with ultrasound or magnetic resonance imaging to confirm an effective ablation zone and avoid ablation of nearby structures such as the carotid baroreceptors. FIGURE 11 is a schematic illustration of a point-ablate cryo-catheter 341 having a cryogenic applicator 340 configured as a metal tip 342. A source of cryogen 344 (e.g. a pressurized canister of nitrous oxide N₂O) is in fluid communication with a supply tube 346 and a valve 345 controls flow. Cryogen in substantially liquid form flows through the supply tube 346 to a distal end of the cryo catheter 341 and through a restriction orifice 348 before exiting into an expansion chamber 347 defined by a cavity in the metal tip 342. The restriction orifice 348 may restrict flow of the cryogen, influencing a pressure differential between the supply tube and the expansion chamber. A large drop in pressure as the cryogen enters the expansion chamber 347 allows the cryogen to change phase from substantially liquid to substantially gas, which is an endothermic reaction absorbing a large amount of heat from surrounding structures. Heat is pulled from tissue through the metal tip 342 and the tissue is cryo-ablated. Control of cryogen flow rate by the valve 345 may control degree of cooling allowing a temporary block and permanent cryo-ablation to be performed with the same device. Gaseous cryogen flows from the expansion chamber 347 through an exhaust lumen 349 and may be exhausted to atmosphere. A point-ablate cryo-catheter 341 may be configured for controlled deflection to allow a physician to deflect or bend a distal region of the cryo-catheter 341 placing the cryo-applicator 342 in contact with a target site. For example, the catheter may comprise a compression-biased structure that bends in a predefined direction upon application of force. For example, a pull wire 352 pulled by an actuator on a handle (not shown) may be connected to a distal end of a laser cut metal tube 350, creating compression of the metal tube causing it to bend in a biased direction. Optionally, the metal tip 342 may be electrically connected to a conductor 354, which may provide electrical communication with an electrical stimulation signal generator 356. A return electrode 358 connected to the electrical stimulation signal generator 356 may be placed on the patient's skin to complete the circuit. This configuration may allow an electrical stimulation signal to be applied to the target site to confirm proximity to a carotid body or safe distance from a baroreceptor (e.g. by producing a physiologic response to the stimulation such as changes in blood pressure, heart rate, ventilation). Other embodiments of cryogenic catheters may be used to ablate a carotid body such as cryo-balloon applicators.

### Intravascular Ultrasound:

An endovascular catheter may alternatively be designed and used to deliver an ultrasound transducer. Intravascular Ultrasound (IVUS) is a medical imaging technology in which a small ultrasound transducer is mounted to a catheter and delivered through a patient's vasculature to provide an ultrasound image from inside the vessel out through blood and into layers of the vessel wall and a short distance beyond. IVUS was developed initially for intravascular imaging of coronary arteries to investigate vascular structures. An IVUS catheter may be applied both extravascularly or intravascularly to the cervical carotid arteries to obtain ultrasound images of carotid arteries and surrounding tissues. IVUS may be a useful tool to locate and visualize a carotid body so an ablation device can be directed to a target site. IVUS may also be useful in imaging a diseased carotid artery bifurcation in order to image plaque that can suggest best approaches to the ablation procedure.

An IVUS catheter can be for example an intravascular ultrasound imaging device Eagle Eye Gold catheter (0.014 guidewire compatible, 2.9 Fr outer shaft diameter) by Volcano Corp, Rancho Cordova, CA.

Data obtained from IVUS may be processed by a computer algorithm to produce useful images, for example to assess material properties, distinguish between plaque and vessel tissue, or identify blood flow. For example, IVUS Virtual Histology^{™} (VH-IVUS) may be used to evaluate the pathological properties of plaque contained within a carotid artery and healthy tissues as well as ablation induced scar tissues. VH-IVUS displays plaque composition within four color categories (e.g. fibrous, fibro-fatty, calcification and necrotic core) being able to offer detailed tissue characterization of soft to hard plaque components.

It is anticipated that improvements can be made to IVUS to make it more usable for carotid artery procedure and CB imaging. A brain embolism protection device may be used in conjunction with IVUS to catch plaque loosened from an artery wall. A catheter-based ultrasound transducer could also be configured to focus ultrasound waves on a target tissue a short depth from a vessel wall to ablate a carotid body.

### Transvascular Ablation:

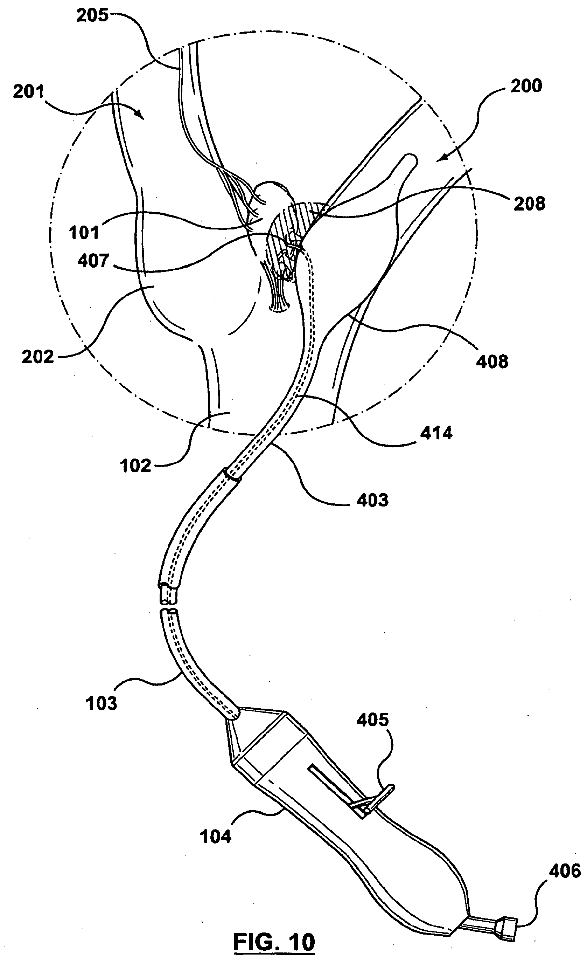

An endovascular catheter may alternatively be designed and used to deliver an agent across a vessel wall to affect target tissue. As shown in FIGURE 10, a transvascular injection catheter 403 has a deployable micro-needle 407 having a lumen 414 in fluid communication through the catheter shaft to an injection hub 406. Once the micro-needle is deployed through a vessel wall (e.g. the wall of an external carotid) a contrast solution may be injected to verify position in a target tissue. An ablative agent, sclerosing agent or a neural disruptive agent may be injected into a target tissue. An example of an agent that may be used to disable sympathetic signaling from a carotid body is Guanethidine, which is known to cause sympathectomy, by inhibiting mitochondrial respiration, and induce an immune response. A temporary neural blockade (e.g. Bupivicane) may be injected to test a response to therapy prior to a more permanent ablative or disruptive injection. Following injection, the micro-needle may be retracted and the catheter removed from the patient. Deployment and retraction of the micro-needle may be achieved with an actuator 405 on a catheter handle. For example, an actuator 405 may be connected to a control wire running through a catheter shaft to a deployable structure 408 at the distal end of the catheter. The deployable structure 408 may be, for example, a deployable mesh, cage, basket, or helix that radially expands to secure the distal end of the catheter in the vessel and causes the micro-needle to protrude. The deployable structure may be an inflatable balloon that is deployed by injecting air or liquid (e.g. saline) into a hub in a handle. Alternatively, a micro-needle may be deployed by a separate control or actuator that advances the micro-needle out of the catheter.

Another example of a transvascular approach is to use an endovascular catheter designed with a deployable micro-needle having an energy delivery element that may be deployed through a vessel wall (e.g. a wall of an external carotid artery) and placed in or proximate to target tissue. The energy delivery element may be, for example, a cryo, RF or electroporation electrode.

### Embolization:

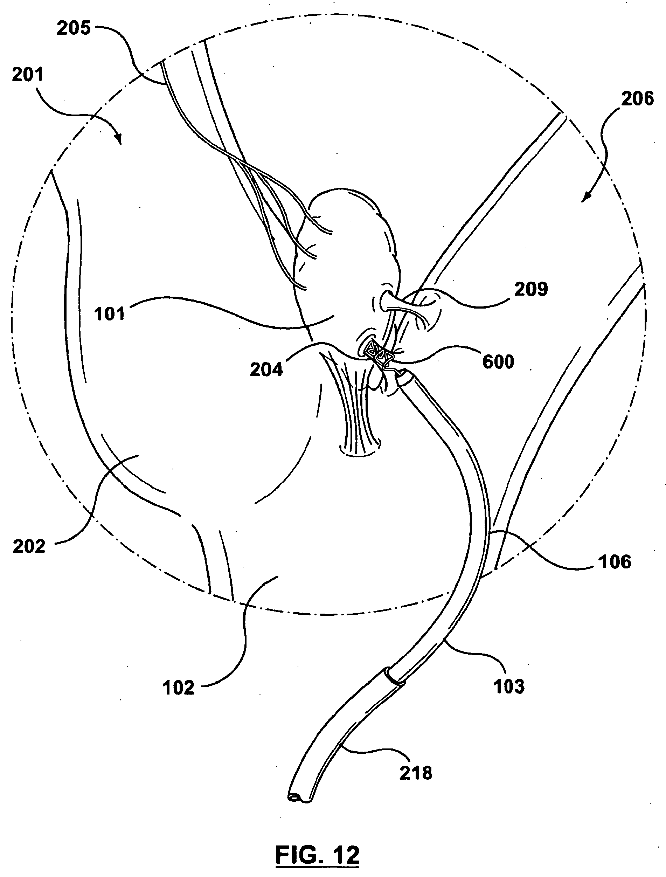

Another embodiment of the present invention, as shown in FIGURE 12 involves embolizing the carotid body 101 by blocking blood flow to the carotid body. For example, a microcatheter may be inserted into small arteries 209 or 204 that feed a carotid body and an implantable occlusion device 600 can be placed in the artery. The implantable occlusion device 600 may be microspheres, filaments, a wire coil, an injectable foam, cement, or hardening composition. Examples of injectable microspheres include 500 to 700 micron spherical polyvinyl alcohol particles (SPVA) such as Contour SE Microspheres made by Boston Scientific, which are commonly used to infarct fibroids. Alternatively 700 to 900µg or even 900 to 1200 µg particles may be used.

An alternative method of embolizing the carotid body may be to ablate or cause stenosis of the blood vessels feeding the carotid body, for example using an endovascular ablation catheter such as a RF catheter to apply thermal energy to the blood vessels 209. This may be accomplished by applying ablative energy (e.g. RF) from a vessel surface such as an external carotid artery. Alternatively, a small catheter may be inserted into a vessel that feeds or drains a carotid body and ablative energy may be applied directly to the walls of these vessels to cause them to close.

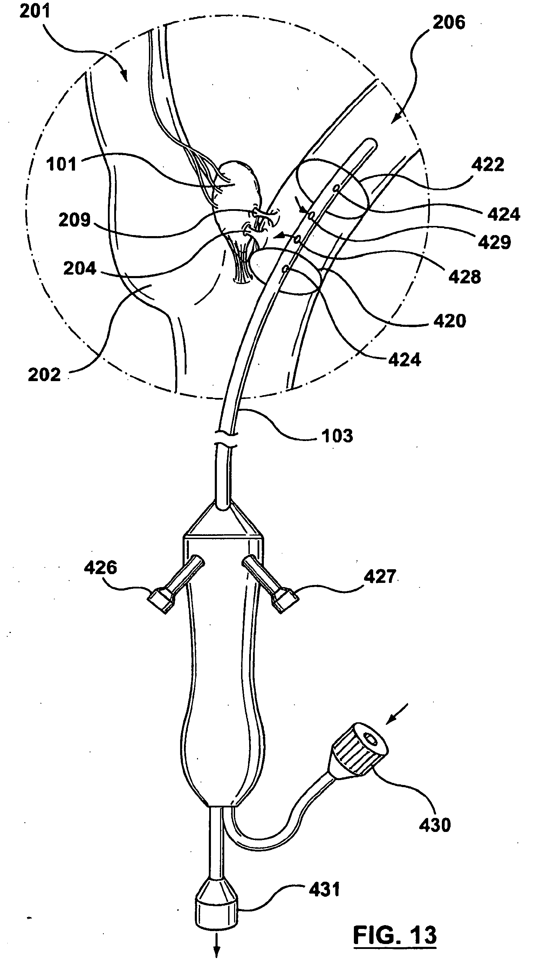

Another alternative embodiment of an embolization device and method, as shown in FIGURE 13, involves a catheter 103 having two inflatable occluding balloons, one proximal 420 and one distal 422. Each of the occlusion balloons may be inflated or expanded by injecting gas or fluid through balloon inflation ports 424 that may be in fluid communication via a lumen through the catheter to one or two inflation hubs 426 and 427 on the proximal end of the catheter for control of inflation of each balloon either independently or concurrently. The catheter would be placed such that the proximal and distal occluding balloons 420 and 422 would occlude the EC 206 proximal and distal to small arteries 204 and 209 feeding a carotid body 101. The catheter may further comprise an injection port 428 and an evacuation port 429 both positioned on the catheter shaft between the proximal and distal occlusion balloons. The injection and evacuation ports are in fluid communication via lumens (not shown) in the catheter shaft to injection 430 and evacuation 431 hubs, respectively. The occlusion balloons create an isolated segment of the vessel (e.g. EC) that is in fluid communication with small vessels connected to a carotid body, and also with lumens traveling through the catheter to and from evacuation 431 and injection 430 hubs. To embolize small vessels feeding a carotid body, an embolization agent (such as those previously described) may be injected through the injection hub 430 which will travel through the catheter and exit the injection port into the isolated segment in the EC and into the feeding vessels 204 and 209. To relieve fluid pressure, blood or injected agent may be evacuated from the isolated segment of the vessel through evacuation port 429 and out of the catheter via the evacuation hub 431. Evacuated fluid may be released to atmospheric pressure or may be pulled out with negative pressure (e.g. using a syringe or vacuum). By occluding an isolated segment in the EC around the vessels that feed the carotid body, injected fluid may be controlled and removed so it does not perfuse downstream through the EC or perfuse into the IC.

Prior to embolization, the same device illustrated in FIGURE 13 may be used to visualize the carotid body on fluoroscopy and confirm correct placement and occlusion of the isolated segment of the vessel. A contrast solution may be injected through the injection hub 430 which will travel through the catheter and exit the injection port in to the isolated space in the vessel and into the carotid body 101.

Additionally or alternatively, a contrast solution may be injected into the isolated space following the removal of the embolizing agent to confirm that the carotid body has been embolized.

### Chemical ablation:

A device similar to the embodiment illustrated in FIGURE 13 may be used in an alternative method to ablate a carotid body using chemical ablation. In this method, a chemical agent is delivered to the isolated segment of the vessel such that it is perfused through vessels feeding the carotid body and into the carotid body. The chemical agent may be, for example, an ablative or neural blocking agent. By occluding an isolated segment in the EC around the vessels that feed the carotid body, injected fluid may be controlled and removed so it does not perfuse downstream through the EC. The isolated segment of the vessel may be flushed by injecting a flushing liquid (e.g. saline) into the isolated segment via the injection port 428 and removing the fluid in the isolated segment via the evacuation port 429.

The same device may be used, prior to chemical ablation, to inject a contrast solution that may be viewed using fluoroscopy to ensure the vessel (e.g. EC) is properly occluded and the small vessels 204 and 209 feeding the carotid body 101 are targeted.

### Guided Percutaneous Procedure:

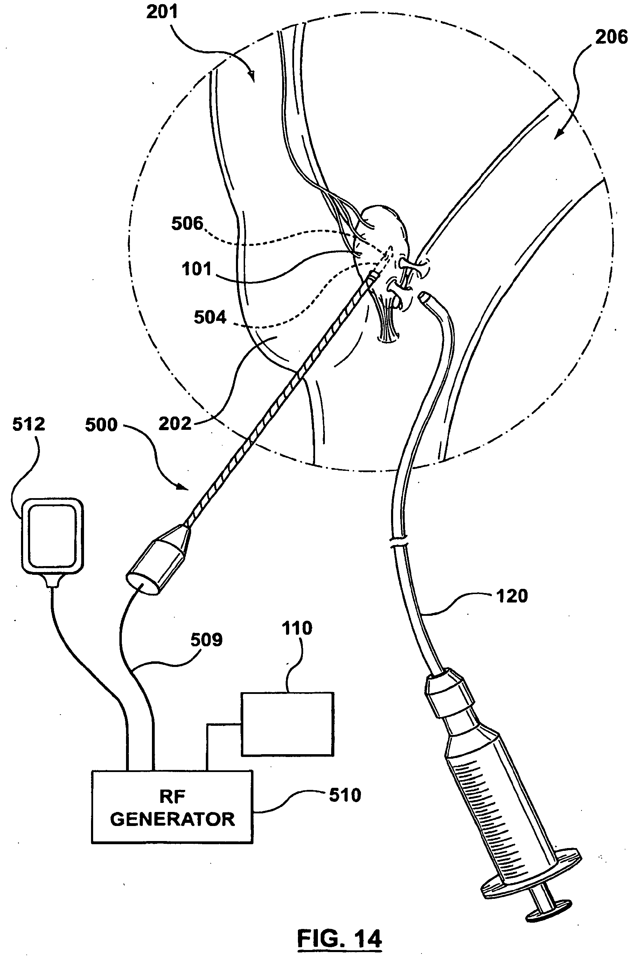

Another embodiment of the present invention, as shown in FIGURE 14 involves providing visualization (e.g. radiographic visualization, Computer Tomography (CT), MRI, or ultrasound) of a carotid body and insertion of a percutaneous ablation device (e.g. a radiofrequency ablation needle 500, a chemical/drug delivery needle, a cryo-probe, a cryo-needle). Visualization of the carotid body or carotid arteries may facilitate safe and effective insertion of a percutaneous ablation device. Several embodiments of visualization methods and devices as well as percutaneous ablation devices are described hereinafter.

In FIGURE 14, the visualization technique shown involves an injection catheter 120 that is used to inject radiographic contrast solution. Contrast may be injected, as shown, in close proximity to arterioles feeding a carotid body so the contrast enters the carotid body allowing it to be seen on a radiograph (e.g. fluoroscope). Alternatively, the contrast may be injected upstream of a carotid body such as in a common carotid artery, to allow visualization of the carotid bifurcation and the internal and external carotid arteries. Even if the carotid body itself is not clearly seen the carotid bifurcation or carotid arteries may be used as landmarks and structures to avoid.

Additionally or optionally, endovascular devices visible with an imaging modality may be placed in the internal jugular vein. It may be desired to avoid damage to the jugular,vein while ablating the carotid body and carotid body nerves. Since the jugular vein and common carotid artery are very close it is beneficial to improve visualization, stability and location of both.

Alternatively, a distal region of an endovascular catheter may be placed in a patient's vasculature proximal to a targeted carotid body to provide assistance during a percutaneous carotid body ablation procedure. Such a catheter may assist a percutaneous ablation procedure by providing additional stability, a fiduciary marker, or thermal protection.

Increased stability could be beneficial to hold the vessel(s) in place while skin is punctured and a percutaneous ablation device is advanced to a target. Increased stability may be provided, for example, by a structure that increases stiffness such as an expanded balloon, mesh or cage.

A fiduciary marker may be, for example, a guidewire, a radiopaque mesh or cage or balloon that allows a vessel proximate to a targeted carotid body to be visualized radiographically and used as a landmark while placing the percutaneous ablation device in a location where a carotid body is expected such as at a lateral side of a carotid bifurcation. Visualizing a proximate vessel may also increase safety by helping a physician avoid puncturing the vessel or ablating too close to the vessel wall.

Thermal protection may benefit a percutaneous ablation procedure that thermally ablates a carotid body (e.g. RF, cryo, direct heat) by maintaining a non-ablative temperature in the vessel wall. For instance, if a RF probe is inserted near or in to a carotid body and a thermal lesion is created to ablate the carotid body, the lesion may expand beyond the carotid body and a carotid artery wall that is close to the RF probe may be in danger of being injured, especially if the RF electrode is very close or touching the carotid artery wall. A thermal protection catheter placed within the artery may actively cool the vessel wall and maintain a non-ablative temperature. Additionally, when a thermally protective element is placed in a carotid sinus 202 it can also protect a carotid baroreceptor from injury. It is understood that a similar thermal protection catheter could be inserted in to the internal jugular vein proximate to the carotid artery to avoid unintentional thermal damage intended to ablate a carotid body.

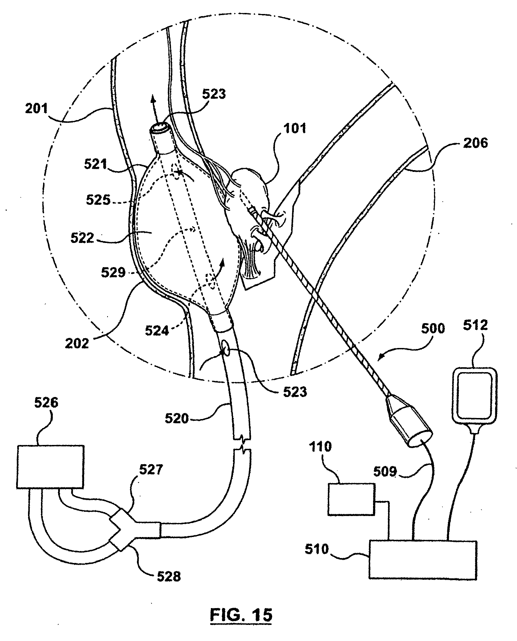

For example, as shown in FIGURE 15, a catheter 520 used to assist a percutaneous ablation procedure comprises an inflatable balloon 521 that is positioned with visual guidance (e.g. fluoroscopy) in a vessel proximal to a targeted carotid body 101 such as an internal 201 or external 206 carotid artery or a carotid bifurcation. The balloon 521 may be made from compliant balloon material and inflated by injecting a contrast solution 522 into the balloon through an inflation lumen 524. Once placed, the balloon may safely occlude the vessel for a short time while a percutaneous ablation procedure is performed. Optionally, the catheter 520 may further comprise a lumen 523 in fluid communication with the proximal and distal sides of the balloon 521 to allow blood flow to continue to flow through the vessel. Optionally, the inflation fluid may comprise a contrast solution that is cooled or is replenished to maintain cool temperature. Inflation fluid may be exhausted through an exhaust lumen 525 while cooled inflation fluid is replenished through the inflation lumen to allow the pressure in the balloon 521 to be approximately maintained. Cooled contrast solution may be supplied and removed by a pump system 526 connected to inlet 527 and outlet 528 ports on a proximal region of the catheter 520. An optional sensor 529 (e.g. temperature sensor, pressure sensor) may be positioned inside the balloon 521 and connected to the pump system 526. A signal from the sensor 529 may be used in a feedback control algorithm to control flow of inflation fluid 522 or temperature in the balloon. A cooled balloon may be used to maintain a non-ablative temperature in the vessel wall while a proximal carotid body is being thermally ablated.

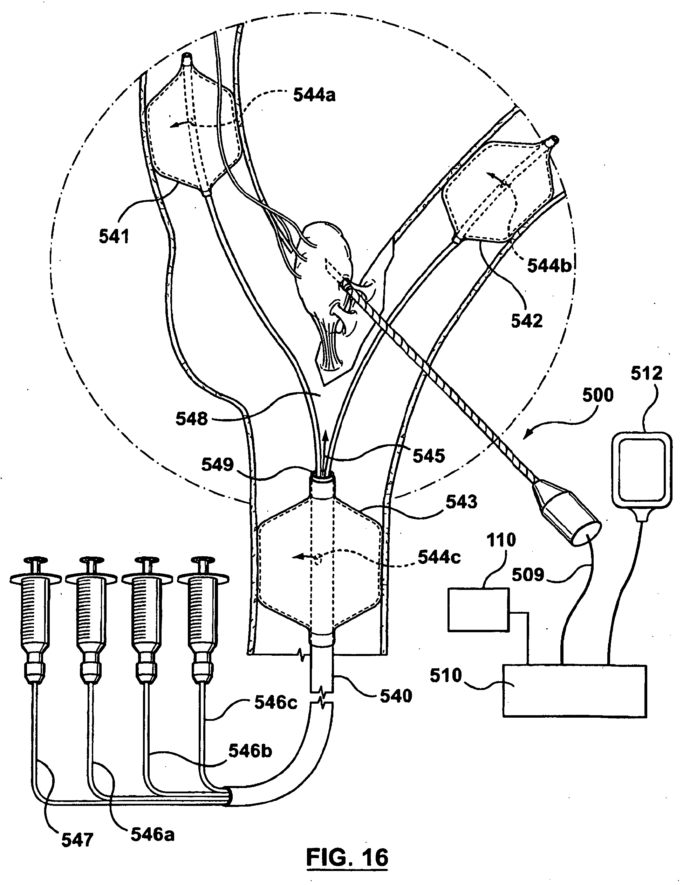

As shown in FIGURE 16, an alternative embodiment of a catheter 540 used to assist a percutaneous ablation procedure comprises multiple inflatable balloons 541 and 542 that are placed to occlude an internal carotid artery 201 and an external carotid artery 206 distal to a carotid bifurcation 200 and carotid body 101 to allow contrast solution injected proximal to the occluding balloons to pool in the arteries, which further allows radiographic visualization while performing a percutaneous carotid body ablation procedure. An optional third balloon 543 may be used to occlude the common carotid artery. Each balloon may be inflated by injecting fluid through inflation lumens 544 in communication with injection ports 546 at the proximal region of the catheter(s). Contrast solution 545 may be injected into the occluded vessel space 548 via a lumen 549 in fluid communication with injection port 547. Optionally, the contrast solution 545 injected into the occluded vessel space 548 may be cooled to provide thermal protection as previously described. Once the occluded vessel space 548 is filled with contrast a percutaneous ablation device may be inserted under fluoroscopy or assisted by ultrasound (e.g. IVUS) to the target carotid body, or space relative to the occluded vessel space where a carotid body is expected to be. An electrical stimulation signal may be delivered to confirm proximity to a carotid body or avoidance of a baroreceptor. Ablation energy may then be delivered. Following ablation, the occluding balloons may be deflated by extracting inflation fluid from the balloons through the inflation lumens. The balloon in the external carotid artery may be deflated first followed by the balloon in the common carotid artery, allowing blood to flow through the external carotid artery before deflating the balloon in the internal carotid artery.

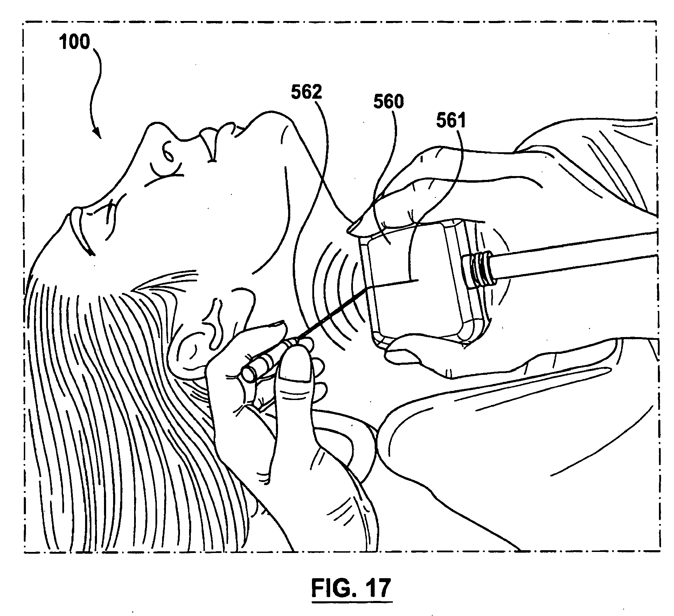

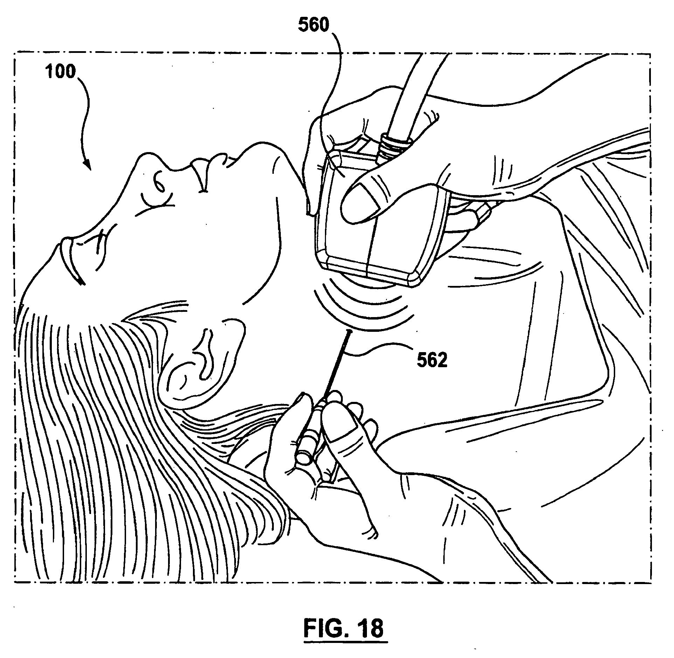

Ultrasound imaging (e.g. sonography) may be a useful technology to visualize a carotid body and surrounding structures (e.g. carotid arteries, jugular vein) to assist the insertion of a percutaneous ablation device or other percutaneous device that may be used in a carotid body ablation procedure (e.g. guide needle wire, micro-needle, needle, stimulation electrode probe). Ultrasound imaging subjects patients and physicians to less radiation and allows soft tissue to be visualized than compared to fluoroscopy and thus may be an alternative technique to contrast injection and radiography for localization of a target. As shown in FIGURE 17 a transducer/transceiver may be placed on an external surface of a patient's skin and aimed toward the patient's targeted carotid body. Gel may be used to aid transducer contact and improve the ultrasound image. The transducer may be connected to an ultrasound system having a display screen. Blood filled vessels will appear hyperechoic and vessel walls will appear bright. Color flow Doppler may additionally be used to visualize vessels. A technique used to locate a carotid body using an ultrasound transducer may comprise placing a transducer 560 on the side of a patient's neck over the common carotid artery 102 to view the common carotid artery as a large round cross-section, which can be further illuminated with Doppler. The transducer 560 may then be slid in a cranial direction while observing the image of the common carotid artery cross-section. As the transducer approaches the carotid bifurcation 200 the image of the cross-section may appear to widen, form a neck or tapering in the center, then divide into two circular cross-sections, which represent the internal 201 and external 206 carotid arteries. The transducer 560 may have a line of sight indicator 561. As shown in FIGURE 17 a percutaneous ablation device 562 (e.g. RF probe) may be inserted along the line of sight indicator 561 to the visualized carotid body, or location where a carotid body is expected to be (e.g. medial side of a saddle of the visualized carotid bifurcation), while safely avoiding the visualized carotid arteries. Optionally the transducer 560 may be placed at additional angles on the patient's neck, for example to obtain a sagittal view of the carotid arteries as shown in FIGURE 18, to provide additional information of location of a percutaneous ablation device 562 relative to arteries or a carotid body. Alternative transducers and techniques may be used to place a percutaneous ablation device 562 safely proximate to a carotid body 101. The percutaneous ablation device 562 may optionally comprise an echogenic design. For example, the device may comprise a textured or grooved surface to increase backscatter of ultrasound waves to the transducer/transceiver, which may improve the visibility of the device.

Alternatively, prior to inserting a percutaneous ablation device to a target site a percutaneous device such as a needle, micro-needle, or electrical stimulation electrode may be placed proximate the target using ultrasound visualization.

It is understood that techniques for using ultrasound to assist percutaneous ablation of a carotid body or carotid body nerves while sparing other nerves, baroreceptors and vessels such as a jugular vein, are applicable to previously described endovascular ablation techniques. It is understood that different modalities of imaging may be combined and that endovascular catheter manipulations do not exclude, but potentially can compliment, percutaneous techniques.

Once the target carotid body or an estimated location of a carotid body such as the medial side of the saddle or bifurcation of carotid artery is identified using visualization, or possibly confirmed using electric stimulation, a percutaneous ablation device may be inserted into or placed proximate to the target. For example, as shown in FIGURE 14 to 16 the percutaneous ablation device may be a radiofrequency ablation needle 500 or cannula. RF ablation may be a desirable energy modality for ablating a carotid body or associated nerves because lesion volume may be controlled. Furthermore, a RF lesion formed proximate a carotid body may effectively ablate a carotid body or associated nerves while safely avoiding undesired or excessive damage of larger vessels such as the common, internal and eternal carotid arteries or jugular veins due to the heat sink in the vessel wall created by significant blood flow. A desirable lesion volume (e.g. about 30 to 900mm³) may be achieved by selecting an RF electrode size (e.g. gauge and length), as well as RF energy delivery parameters (e.g. power, duration, and ramp slope). The RF ablation needle may be, for example equal to or smaller than about a 16 gauge needle (e.g. 20 gauge, 22 gauge) and may be electrically insulated along its shaft 502 with an electrically exposed distal tip 504 that is equal to or less than about 10mm long (e.g. 2 to 5mm). The RF ablation needle 500 may include a temperature sensor 506 in the distal tip 504. The RF ablation needle and temperature sensor may be connected to a computerized RF generator 510 via connection wires 509. A reference electrode 512 may be placed on the patient's skin to complete the RF circuit. The computerized RF generator 510 can control the delivery of RF energy to the RF ablation needle 500 using a feedback control algorithm 110 incorporating temperature data from the temperature sensor 506. Optionally, a RF ablation needle may have a blunt or rounded tip to reduce the risk of puncturing a carotid artery or jugular vein. A blunt tip RF ablation needle may be inserted through a small incision made with a scalpel or introducer needle in a patient's skin. Alternatively, a RF probe system may comprise a cannula with a square cut tip and a sharp trocar (e.g. pencil point, or beveled tip) that protrudes from the cannula to puncture through tissues such as the skin or carotid fascia. The sharp trocar may be replaced with a blunt tip trocar, stimulation electrode, or RF electrode when approaching or maneuvering proximate a carotid artery or carotid body (e.g. target site). Yet another alternative to a sharp trocar is to use a RF perforation electrode protruding from the cannula. RF perforation (e.g. perforation through electroporation) may be performed by delivering RF perforation parameters provided by a RF generator. For example, the RF energy may be configured to operate at high impedance (e.g. 2000-6000Ω), low power (e.g. 5-25W), high voltage (e.g. 150-180V) at short pulses (e.g. 0.25-3 seconds). Such RF parameters delivered through a relatively small electrode may cause tissue cells to rupture allowing the electrode to gently push through tissue. A benefit of passing through tissue such as carotid fascia using RF perforation is that less physical force is required and tenting (a phenomenon of a tough tissue resisting puncture of a sharp object and deforming to the object in a tent shape until enough force is applied to break through the tissue) is greatly reduced. Thus a risk of puncturing through a carotid fascia with a sharp device and inadvertently advancing the sharp device too far or uncontrolled into a carotid artery is greatly reduced. RF perforation energy may be turned off or the RF perforation electrode may be replaced by a blunt tip or sharp tip trocar or electrode in the cannula to pass the device through softer tissue.

Additionally, once the percutaneous ablation device is inserted to a desired location and prior to applying ablative energy, stimulation current can be applied to the carotid body to confirm the location of the ablation device. For example stimulation current can be current known to excite afferent sympathetic nerve fibers (e.g. 20Hz, 100µs pulse, 1-10mA). A sympathetic physiological response (e.g. increased blood pressure, respiration or heart rate) could indicate that the instrument is at the right location and ablation energy may then be applied. Negative responses that will suggest that a different location can be sought will be reduction of HR and BP (caused by baroreflex or vagal nerve stimulation), protrusion of the tongue, or twitch of facial or throat muscles (caused by hypoglossal nerve stimulation), or expected clinical signs of sympathetic activation (caused by sympathetic trunk stimulation).

Local anesthesia may interfere with a study of reflexes (e.g. electrical or chemical stimulation of a chemoreflex) by disabling all nerves in the area of infiltration by local anesthetic. Sedation with IV anesthetic or general anesthesia can be used to manage patient discomfort while permitting physiologic response to chemoreflex stimulation. Alternatively, local anesthesia can be implemented after the reflexes and location of carotid body is confirmed with a very small needle or electrical stimulation electrode (e.g. 20 to 25 gauge), which may be less painful to the patient than a larger percutaneous ablation device. For example, a small electrical stimulation electrode may be configured with a lumen and be inserted to a target site, stimulation may be applied and, upon confirmation of proximity to a carotid body via physiological response monitoring, local anesthetic and optionally contrast solution may be injected through the small electrical stimulation electrode lumen, which may allow a larger percutaneous ablation device to be inserted with minimal pain.

Another embodiment of confirming proximity to a carotid body prior to applying ablative energy makes use of a high concentration of somatostatin receptor sites in carotid bodies. A small amount of radioactive agent with a short half-life such as Indium-111 labeled somatostatin or commercially available Indium In-111 pentetreotide can be injected into a patient's blood stream or directly into a carotid artery. Indium In-111 pentetreotide is currently used for the scintigraphic localization of primary and metastatic neuroendocrine tumors bearing somatostatin receptors. CT scan scintigraphy is expected to allow for a high degree of accuracy in localizing carotid body. This or similar substances can be injected intravenously as commonly used in clinical practice. In addition, previously described techniques for EC occlusion (see FIGURE 13) with balloons can assist injection of localization agents such as iodine based radiocontrast, gadolinium or In-111 pentetreotide as well as to reduce the amount of radioactive material required. The occlusion or partial occlusion technique will increase dwell time and penetration of such agents into the carotid body.

Alternatively, a different form of percutaneous ablation device may be used to deliver an ablative energy to ablate targeted tissue. For example, a cryogenic probe, thermal probe, or needle for injection of a chemical or ablating or sclerosing agent may be introduced through the skin of a patient's neck proximate to the carotid body as a percutaneous ablation technique. Alternatively, a cannula (e.g. a hollow needle) may be used to provide percutaneous access through tissue to introduce an ablation probe or catheter with electrodes or a drug infusion catheter.

Another embodiment of a percutaneous approach may involve using magnetic resonance imaging (MRI) to visualize a targeted carotid body and carotid arteries. A MRI antenna may be placed in the adjacent artery or vein or on the external surface of a patient's neck proximate to a patient's carotid body to increase resolution. A percutaneous ablation device made from MRI compatible material such as Nitinol may be used to inject an ablative solution or to apply RF energy. Since RF generators are high impedance circuits they do not interfere with MRI.

### Extracorporeal:

Another alternative embodiment may ablate a carotid body using energy applied from outside the patient's body. For example, systems employing extracorporeal High Intensity Focused Ultrasound (HIFU) or stereotactic radiotherapy may be used to focus ablative energy in targeted tissue. The targeted tissue may be identified and tracked using a non-invasive technology such as CT, MRI or sonography. Alternatively, a technique for visualization/identification that is described later may be employed.

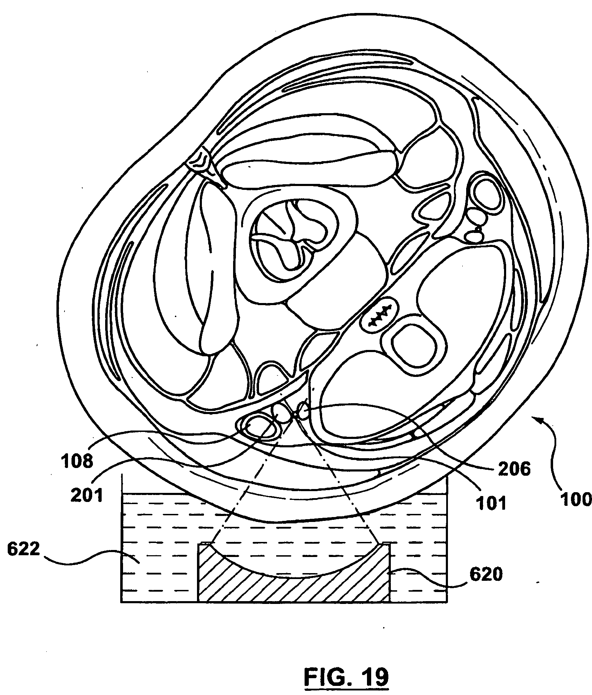

HIFU is a technology involving an ultrasound transducer (or array of transducers) positioned external to the body that focuses ultrasound beams, via a lens, a curved shape of the transducer, or dynamic control of a phased array of transducers), on a small volume of tissue a given depth from the surface. The focused beams heat tissue in the focal zone to an ablation temperature (e.g. 65 to 85°C). As shown in FIGURE 19, a HIFU transducer 620 may be placed external a patient 100 and aimed at a carotid body 101. Ultrasound beams travel through a conductive medium 622 such as water and through the patient 100 where they are focused on a target site (e.g. carotid body) while sparing significant undesired damage to other tissues (e.g. jugular vein 108, internal carotid artery 201, external carotid artery 206). Locating the target site may be assisted using imaging technology such as MRI, CT or sonography. Optionally, prior to, or during ablation the transducer 620 may deliver ultrasound waves that do not heat tissue but create agitation. This could be used to stimulate the carotid body and confirm that the transducer is aimed at the correct location through monitoring physiological reactions. Following ablation agitation can confirm that the carotid body is ablated if it is no longer stimulated. Physiological monitors (e.g. heart rate monitor, blood pressure monitor, blood flow monitor, MSNA monitor) may communicate with a computerized HIFU generator to provide feedback information in response to stimulation. If a physiological response correlates to a given stimulation the computerized HIFU generator may provide an indication of a positive confirmation.

### Methods of Therapy:

A method in accordance with a particular embodiment includes ablating at least one of a patient's carotid bodies based at least in part on identifying the patient as having a sympathetically mediated disease such as cardiac, metabolic, or pulmonary disease such as hypertension (e.g. refractory hypertension), congestive heart failure (CHF), or dyspnea.

A procedure may include diagnosis, selection based on diagnosis, further screening (e.g. baseline assessment of chemosensitivity), treating a patient based at least in part on diagnosis or further screening via a chemoreceptor (e.g. carotid body) ablation procedure such as one of the embodiments disclosed. Additionally, following ablation a method of therapy may involve conducting a post-ablation assessment to compare with the baseline assessment and making decisions based on the assessment (e.g. adjustment of drug therapy, re-treat in new position or with different parameters, or ablate a second chemoreceptor if only one was previously ablated).

A chemoreceptor ablation procedure may comprise the following steps or a combination thereof: patient sedation, locating a target peripheral chemoreceptor, visualizing a target peripheral chemoreceptor, confirming a target ablation site is or is proximate a peripheral chemoreceptor, confirming a target ablation site is safely distant from a baroreceptor or its nerves, providing stimulation (e.g. electrical, mechanical, chemical) to a target site or target peripheral chemoreceptor prior to, during or following an ablation step, monitoring physiological responses to said stimulation, anesthetizing a target site, protecting the brain from potential embolism, thermally protecting a proximate baroreceptor, ablating a target site or peripheral chemoreceptor, monitoring ablation parameters(e.g. temperature, impedance, blood flow in a carotid artery), confirming a reduction of chemoreceptor activity (e.g. chemosensitivity, HR, blood pressure, ventilation, sympathetic nerve activity) during or following an ablation step, removing an ablation device, conducting a post-ablation assessment, repeating any steps of the chemoreceptor ablation procedure on another peripheral chemoreceptor in the patient.

Patient screening, as well as post-ablation assessment may include physiological tests or gathering of information, for example, chemoreflex sensitivity, central sympathetic nerve activity, heart rate, heart rate variability, blood pressure, ventilation, production of hormones, peripheral vascular resistance, blood pH, blood PCO2, degree of hyperventilation, peak VO2, VE/VCO2 slope. Directly measured maximum oxygen uptake (more correctly pVO2 in heart failure patients) and index of respiratory efficiency VE/VCO2 slope has been shown to be a reproducible marker of exercise tolerance in heart failure and provide objective and additional information regarding a patient's clinical status and prognosis.

A method of therapy may include electrical stimulation of a target region, using a stimulation electrode, to confirm proximity to a carotid body. For example, a stimulation signal having a 1-10 milliamps (mA) pulse train at about 20 to 40Hz with a pulse duration of 50 to 500 microseconds (µs) that produces a positive carotid body stimulation effect may indicate that the stimulation electrode is within sufficient proximity to the carotid body or nerves of the carotid body to effectively ablate it. A positive carotid body stimulation effect could be increased blood pressure, heart rate, or ventilation concomitant with application of the stimulation. These variables could be monitored, recorded, or displayed to help assess confirmation of proximity to a carotid body. A catheter-based technique, for example, may have a stimulation electrode proximal to the energy delivery element used for ablation. Alternatively, the energy delivery element itself may also be used as a stimulation electrode. FIGURE 7 can illustrate an embodiment of this concept where a radiofrequency electrode 207 may also be used to deliver a stimulation signal. In this case, the RF generator 210 or a separate stimulation generator may provide the stimulation signal to the electrode 207. Alternatively, an energy delivery element that delivers a form of ablative energy that is not electrical, such as a cryogenic ablation applicator, may be configured to also deliver an electrical stimulation signal as described earlier. Yet another alternative comprises a stimulation electrode that is distinct from an ablation element. For example, during a surgical procedure a stimulation probe can be touched to a suspected carotid body that is surgically exposed. A positive carotid body stimulation effect could confirm that the suspected structure is a carotid body and ablation can commence. Physiological monitors (e.g. heart rate monitor, blood pressure monitor, blood flow monitor, MSNA monitor) may communicate with a computerized stimulation generator, which may also be an ablation generator, to provide feedback information in response to stimulation. If a physiological response correlates to a given stimulation the computerized generator may provide an indication of a positive confirmation.

Alternatively or in addition a drug known to excite the chemo sensitive cells of the carotid body can be injected directly into the carotid artery or given systemically into patients vein or artery in order to elicit hemodynamic or respiratory response. Examples of drugs that may excite a chemoreceptor include nicotine, atropine, Doxapram, Almitrine, hyperkalemia, Theophylline, adenosine, sulfides, Lobeline, Acetylcholine, ammonium chloride, methylamine, potassium chloride, anabasine, coniine, cytosine, acetaldehyde, acetyl ester and the ethyl ether of i-methylcholine, Succinylcholine, Piperidine, monophenol ester of homo-iso-muscarine and acetylsalicylamides, alkaloids of veratrum, sodium citrate, adenosinetriphosphate, dinitrophenol, caffeine, theobromine, ethyl alcohol, ether, chloroform, phenyldiguanide, sparteine, coramine (nikethamide), metrazol (pentylenetetrazol), iodomethylate of dimethylaminomethylenedioxypropane, ethyltrimethylammoniumpropane, trimethylammonium, hydroxytryptamine, papaverine, neostigmine, acidity.

A method of therapy may further comprise applying electrical or chemical stimulation to the target area or systemically following ablation to confirm a successful ablation. Heart rate, blood pressure or ventilation may be monitored for change or compared to the reaction to stimulation prior to ablation to assess if the targeted carotid body was ablated. Post-ablation stimulation may be done with the same apparatus used to conduct the pre-ablation stimulation. Physiological monitors (e.g. heart rate monitor, blood pressure monitor, blood flow monitor, MSNA monitor) may communicate with a computerized stimulation generator, which may also be an ablation generator, to provide feedback information in response to stimulation. If a physiological response correlated to a given stimulation is reduced following an ablation compared to a physiological response prior to the ablation, the computerized generator may provide an indication ablation efficacy or possible procedural suggestions such as repeating an ablation, adjusting ablation parameters, changing position, ablating another carotid body or chemosensor, or concluding the procedure.

### Visualization:

An optional step of visualizing internal structures (e.g. carotid body or surrounding structures) may be accomplished using one or more non-invasive imaging modalities (e.g. fluoroscopy, radiography, arteriography, CT, MRI, sonography) or minimally invasive techniques (e.g. IVUS, endoscopy, optical coherence tomography). A visualization step may be performed as part of a patient assessment, prior to an ablation procedure to assess risks and location of anatomical structures, during an ablation procedure to help guide an ablation device, or following an ablation procedure to assess outcome (e.g. efficacy of the ablation).

FIGURE 13 shows a schematic view of blood vessels, such as small arteries 204 and 209 perfusing the glomus, and the carotid sinus nerve 205. The carotid sinus nerve 205 carries signals from chemoreceptors in the carotid body 101 and baroreceptors in.the carotid sinus 202.

Due to proximity to the wall of the CC and consistent (expected in at least 80% of the cases) position of the carotid body in humans the procedure may be guided visually using fluoroscopy assisted by radiographic contrast injections into the CC, IC, or EC.

Endovascular (for example transfemoral) arteriography of the common carotid and then selective arteriography of the internal and external carotids may be used to determine a position of a catheter tip at a CC bifurcation. Additionally, ostia of glomic arteries (these arteries may be up to 4 mm long and arise directly from the main parent artery) can be identified by dragging the dye injection catheter and releasing small amounts ("puffs") of dye. If a glomic artery is identified it can be cannulated by a guide wire and possibly further cannulated by small caliber catheter. Direct injection of dye into glomic arteries can further assist the interventionalist in the ablation procedure. It is appreciated that the feeding glomic arteries are small and microcatheters may be needed to cannulate them.

Alternatively, ultrasound visualization may allow a physician to see the carotid arteries and even the carotid body. Another method for visualization may consist of inserting a small needle (e.g. 22 Gauge) with computer tomography (CT) guidance into or toward the carotid body. A wire or needle can be left in place as a fiducial guide, or contrast can be injected into the carotid body. Runoff of contrast to the jugular vein may confirm that the target is achieved.

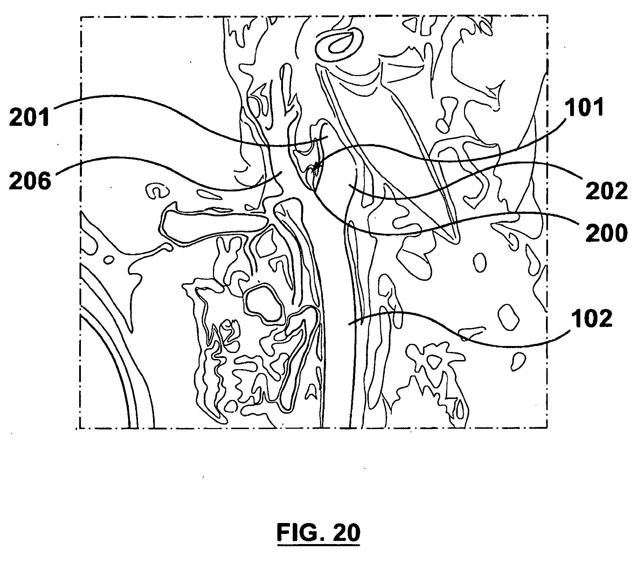

Computer Tomography (CT) may also be used to aid in identifying a carotid body. FIGURE 20 (Nguyen R.P. Carotid Body Detection on CT Angiography, AM J Neuroradiology 32:1096-99, Jun-Jul 2011) is a CT image, performed with a B20 kernel at a 2mm thickness at 2mm increments, of an oblique sagittal view of subject's neck showing a carotid body at a carotid bifurcation. Such imaging could be used to help guide an ablation device to a carotid body or provide targeting for an extracorporeal ablation procedure (e.g. HIFU, stereotactic radiotherapy).

Ultrasound visualization (e.g. sonography) is an ultrasound-based imaging technique used for visualizing subcutaneous body structures including blood vessels and surrounding tissues. Doppler ultrasound uses reflected ultrasound waves to identify and display blood flow through a vessel. Operators typically use a hand-held transducer/transceiver placed directly on a patient's skin and aimed inward directing ultrasound waves through the patient's tissue. Ultrasound may be used to visualize a patient's carotid body to help guide an ablation device.

Visualization and navigation steps may comprise multiple imaging modalities (e.g. CT, fluoroscopy, ultrasound) superimposed digitally to use as a map for instrument positioning. Superimposing borders of great vessels such as carotid arteries can be done to combine images.

Responses to stimulation at different coordinate points can be stored digitally as a 3-dimensional or 2-dimenisional orthogonal plane map. Such an electric map of the carotid bifurcation showing points, or point coordinates that are electrically excitable such as baroreceptors, baroreceptor nerves, chemoreceptors and chemoreceptor nerves can be superimposed with an image (e.g. CT, fluoroscopy, ultrasound) of vessels. This can be used to guide the procedure, and identify target areas and areas to avoid.

In addition, as noted above, it should be understood that a device providing therapy can also be used to locate a carotid body as well as to provide various stimuli (electrical, chemical, other) to test a baseline response of the CBC/CSB and measure changes in these responses after therapy or a need for additional therapy to achieve the desired physiological and clinical effects.

### Patient Selection and Assessment:

In an embodiment, a procedure may comprise assessing a patient to be a plausible candidate for carotid body ablation. Such assessment may involve diagnosing a patient with a sympathetically mediated disease (e.g. MSNA microneurography, measure of cataclomines in blood or urine, heart rate, or low/high frequency analysis of heart rate variability may be used to assess sympathetic tone). Patient assessment may further comprise other patient selection criteria, for example indices of high carotid body activity (i.e. carotid body hypersensitivity or hyperactivity) such as a combination of hyperventilation and hypocarbia at rest, high carotid body nerve activity (e.g. measured directly), incidence of periodic breathing, dyspnea, central sleep apnea elevated brain natriuretic peptide, low exercise capacity, having cardiac resynchronization therapy, atrial fibrillation, ejection fraction of the left ventricle, using beta blockers or ACE inhibitors.

Patient assessment may further involve selecting patients with high peripheral chemosensitivity (e.g. a respiratory response to hypoxia normalized to the desaturation of oxygen greater than or equal to about 0.7 1/min/min SpO₂), which may involve characterizing a patient's chemoreceptor sensitivity, reaction to temporarily blocking carotid body chemoreflex, or a combination thereof.

Although there are many ways to measure chemosensitivity they can be divided into (a) active provoked response and (b) passive monitoring. Active tests can be done by inducing intermittent hypoxia (such as by taking breaths of nitrogen or CO₂ or combination of gases) or by rebreathing air into and from a 4 to 10 liter bag. For example: a hypersensitive response to a short period of hypoxia measured by increase of respiration or heart rate may provide an indication for therapy. Ablation or significant reduction of such response could be indicative of a successful procedure. Also, electrical stimulation, drugs and chemicals (e.g. dopamine, lidocane) exist that can block or excite a carotid body when applied.

The location and baseline function of the desired area of therapy (including the carotid and aortic chemoreceptors and baroreceptors and corresponding nerves) may be determined prior to therapy by application of stimuli to the carotid body or other organs that would result in an expected change in a physiological or clinical event such as an increase or decrease in SNS activity, heart rate or blood pressure. These stimuli may also be applied after the therapy to determine the effect of the therapy or to indicate the need for repeated application of therapy to achieve the desired physiological or clinical effect(s). The stimuli can be either electrical or chemical in nature and can be delivered via the same or another catheter or can be delivered separately (such as injection of a substance through a peripheral IV to affect the CBC that would be expected to cause a predicted physiological or clinical effect).

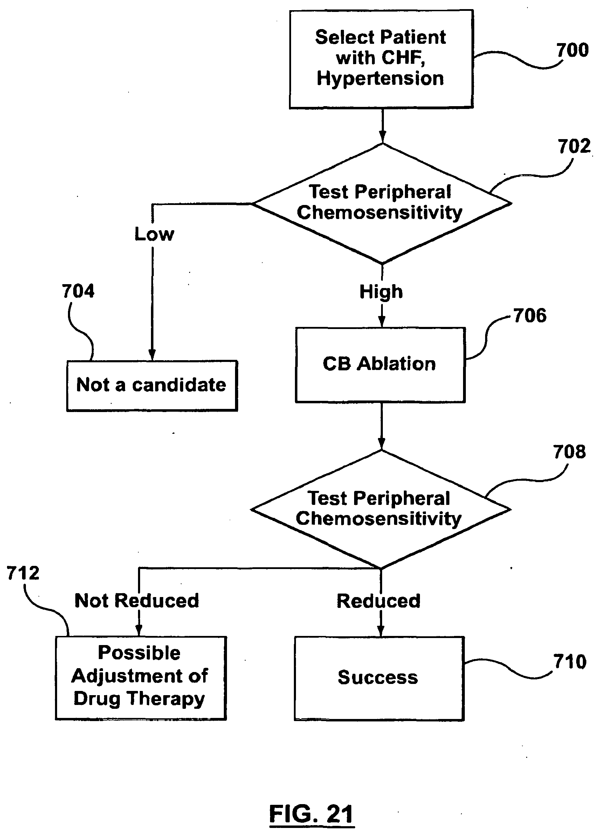

As shown in FIGURE 21, a baseline stimulation test may be performed to select patients that may benefit from a carotid body ablation procedure. For example, patients with a high peripheral chemosensitivity gain (e.g. greater than or equal to about two standard deviations above an age matched general population chemosensitivity, or alternatively above a threshold peripheral chemosensitivity to hypoxia of 0.5 or 0.7 ml/min/%O2) may be selected for a carotid body ablation procedure. A prospective patient suffering from a cardiac, metabolic, or pulmonary disease (e.g. hypertension, CHF, diabetes) may be selected 700. The patient may then be tested to assess a baseline peripheral chemoreceptor sensitivity (e.g. minute ventilation, tidal volume, ventilator rate, heart rate, or other response to hypoxic or hypercapnic stimulus) 702. Baseline peripheral chemosensitivity may be assessed using tests known in the art which involve inhalation of a gas mixture having reduced O₂ content (e.g. pure nitrogen, CO₂, helium, or breathable gas mixture with reduced amounts of O₂ and increased amounts of CO₂) or rebreathing of gas into a bag. Concurrently, the patient's minute ventilation or initial sympathetically mediated physiologic parameter such as minute ventilation or HR may be measured and compared to the O₂ level in the gas mixture. Tests like this may elucidate indices called chemoreceptor set-point and gain. These indices are indicative of chemoreceptor sensitivity. If the patient's chemosensitivity is not assessed to be high (e.g. less than about two standard deviations of an age matched general population chemosensitivity, or other relevant numeric threshold) then the patient may not be a suitable candidate for a carotid body ablation procedure 704. Conversely, a patient with chemoreceptor hypersensitivity (e.g. greater than or equal to about two standard deviations above normal) may proceed to have a carotid body ablation procedure 706. Following a carotid body ablation procedure the patient's chemosensitivity may optionally be tested again 708 and compared to the results of the baseline test 702. The second test 708 or the comparison of the second test to the baseline test may provide an indication of treatment success 710 or suggest further intervention 712 such as possible adjustment of drug therapy, repeating the carotid body ablation procedure with adjusted parameters or location, or performing another carotid body ablation procedure on a second carotid body if the first procedure only targeted one carotid body. It may be expected that a patient having chemoreceptor hypersensitivity or hyperactivity may return to about a normal sensitivity or activity following a successful carotid body ablation procedure.

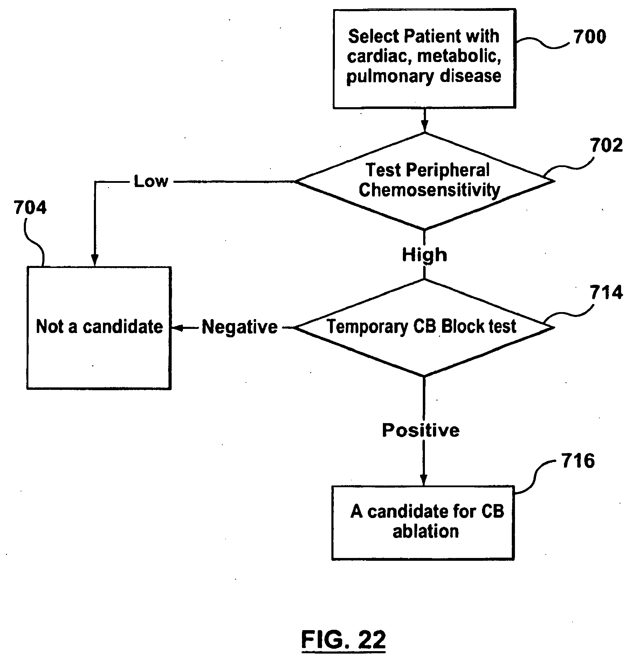

An alternative protocol for selecting a patient for a carotid body ablation procedure is shown in FIGURE 22. Patients with high peripheral chemosensitivity or carotid body activity (e.g. ≥ about 2 standard deviations above normal) alone or in combination with other clinical and physiologic parameters may be particularly good candidates for carotid body ablation therapy if they further respond positively to temporary blocking of carotid body activity. As shown in FIGURE 22, a prospective patient suffering from a cardiac, metabolic, or pulmonary disease may be selected 700 to be tested to assess the baseline peripheral chemoreceptor sensitivity 702. A patient without high chemosensitivity may not be a plausible candidate 704 for a carotid body ablation procedure. A patient with a high chemosensitivity may be given a further assessment that temporarily blocks a carotid body chemoreflex 714. For example a temporary block may be done chemically, for example using a chemical such as intravascular dopamine or dopamine-like substances, intravascular alpha-2 adrenergic agonists, oxygen, in general alkalinity, or local or topical application of atropine externally to the carotid body. A patient having a negative response to the temporary carotid body block test (e.g. sympathetic activity index such as respiration, HR, heart rate variability, MSNA, vasculature resistance, etc. is not significantly altered) may be a less plausible candidate 704 for a carotid body ablation procedure. Conversely, a patient with a positive response to the temporary carotid body block test (e.g. respiration or index of sympathetic activity is altered significantly) may be a more plausible candidate for a carotid body ablation procedure 716.

There are a number of potential ways to conduct a temporary carotid body block test 714. Hyperoxia (e.g. higher than normal levels of PO₂) for example, is known to partially block (about a 50%) or reduce afferent sympathetic response of the carotid body. Thus, if a patient's sympathetic activity indexes (e.g. respiration, HR, HRV, MSNA) are reduced by hyperoxia (e.g. inhalation of higher than normal levels of O₂) for 3-5 minutes, the patient may be a particularly plausible candidate for carotid body ablation therapy. A sympathetic response to hyperoxia may be achieved by monitoring minute ventilation (e.g. reduction of more than 20-30% may indicate that a patient has carotid body hyperactivity). To evoke a carotid body response, or compare it to carotid body response in normoxic conditions, CO₂ above 3-4% may be mixed into the gas inspired by the patient (nitrogen content will be reduced) or another pharmacological agent can be used to invoke a carotid body response to a change of CO₂, pH or glucose concentration. Alternatively, "withdrawal of hypoxic drive" to rest state respiration in response to breathing a high concentration O₂ gas mix may be used for a simpler test.

An alternative temporary carotid body block test involves administering a sub-anesthetic amount of anesthetic gas halothane, which is known to temporarily suppress carotid body activity. Furthermore, there are injectable substances such as dopamine that are known to reversibly inhibit the carotid body. However, any substance, whether inhaled, injected or delivered by another manner to the carotid body that affects carotid body function in the desired fashion may be used.

Another alternative temporary carotid body block test involves application of cryogenic energy to a carotid body (i.e. removal of heat). For example, a carotid body or its nerves may be cooled to a temperature range between about -15°C to 0°C to temporarily reduce nerve activity or blood flow to and from a carotid body thus reducing or inhibiting carotid body activity.

An alternative method of assessing a temporary carotid body block test may involve measuring pulse pressure. Noninvasive pulse pressure devices such as Nexfin (made by BMEYE, based in Amsterdam, The Netherlands) can be used to track beat-to-beat changes in peripheral vascular resistance. Patients with hypertension or CHF may be sensitive to temporary carotid body blocking with oxygen or injection of a blocking drug. The peripheral vascular resistance of such patients may be expected to reduce substantially in response to carotid body blocking. Such patients may be good candidates for carotid body ablation therapy.

Yet another index that may be used to assess if a patient may be a good candidate for carotid body ablation therapy is increase of baroreflex, or baroreceptor sensitivity, in response to carotid body blocking. It is known that hyperactive chemosensitivity suppresses baroreflex. If carotid body activity is temporarily reduced the carotid sinus baroreflex (baroreflex sensitivity (BRS) or baroreflex gain) may be expected to increase. Baroreflex contributes a beneficial parasympathetic component to autonomic drive. Depressed BRS is often associated with an increased incidence of death and malignant ventricular arrhythmias. Baroreflex is measurable using standard non-invasive methods. One example is spectral analysis of RR interval of ECG and systolic blood pressure variability in both the high- and low-frequency bands. An increase of baroreflex gain in response to temporary blockade of carotid body can be a good indication for permanent therapy. Baroreflex sensitivity can also be measured by heart rate response to a transient rise in blood pressure induced by injection of phenylephrine.

An alternative method involves using an index of glucose tolerance to select patients and determine the results of carotid body blocking or removal in diabetic patients. There is evidence that carotid body hyperactivity contributes to progression and severity of metabolic disease.

In general, a beneficial response can be seen as an increase of parasympathetic or decrease of sympathetic tone in the overall autonomic balance. For example, Power Spectral Density (PSD) curves of respiration or HR can be calculated using nonparametric Fast Fourier Transform algorithm (FFT). FFT parameters can be set to 256-64k buffer size, Hamming window, 50% overlap, 0 to 0.5 or 0.1 to 1.0 Hz range. HR and respiratory signals can be analyzed for the same periods of time corresponding to (1) normal unblocked carotid body breathing and (2) breathing with blocked carotid body.

Power can be calculated for three bands: the very low frequency (VLF) between 0 and 0.04 Hz, the low frequency band (LF) between 0.04-0.15 Hz and the high frequency band (HF) between 0.15-0.4 Hz. Cumulative spectral power in LF and HF bands may also be calculated; normalized to total power between 0.04 and 0.4 Hz (TF=HF+LF) and expressed as % of total. Natural breathing rate of CHF patient, for example, can be rather high, in the 0.3-0.4 Hz range.

The VLF band may be assumed to reflect periodic breathing frequency (typically 0.016 Hz) that can be present in CHF patients. It can be excluded from the HF/LF power ratio calculations.

The powers of the LF and HF oscillations characterizing heart rate variability (HRV) appear to reflect, in their reciprocal relationship, changes in the state of the sympathovagal (sympathetic to parasympathetic) balance occurring during numerous physiological and pathophysiological conditions. Thus, increase of HF contribution in particular can be considered a positive response to carotid body blocking.

Another alternative method of assessing carotid body activity comprises nuclear medicine scanning, for example with ocretide, somatostatin analogues, or other substances produced or bound by the carotid body.

Furthermore, artificially increasing blood flow may reduce carotid body activation. Conversely artificially reducing blood flow may stimulate carotid body activation. This may be achieved with drugs know in the art to alter blood flow.

There is a considerable amount of scientific evidence to demonstrate that hypertrophy of a carotid body often accompanies disease. A hypertrophied (i.e. enlarged) carotid body may further contribute to the disease. Thus identification of patients with enlarged carotid bodies may be instrumental in determining candidates for therapy. Imaging of a carotid body may be accomplished by angiography performed with radiographic, computer tomography, or magnetic resonance imaging.

It should be understood that the available measurements are not limited to those described above. It may be possible to use any single or a combination of measurements that reflect any clinical or physiological parameter effected or changed by either increases or decreases in carotid body function to evaluate the baseline state, or change in state, of a patient's chemosensitivity.

There is a considerable amount of scientific evidence to demonstrate that hypertrophy of a carotid body often accompanies disease. A hypertrophied or enlarged carotid body may further contribute to the disease. Thus identification of patients with enlarged carotid bodies may be instrumental in determining candidates for therapy.

Further, it is possible that although patients do not meet a preselected clinical or physiological definition of high peripheral chemosensitivity (e.g. greater than or equal to about two standard deviations above normal), administration of a substance that suppresses peripheral chemosensitivity may be an alternative method of identifying a patient who is a candidate for the proposed therapy. These patients may have a different physiology or co-morbid disease state that, in concert with a higher than normal peripheral chemosensitivity (e.g. greater than or equal to normal and less than or equal to about 2 standard deviations above normal), may still allow the patient to benefit from carotid body ablation. The proposed therapy may be at least in part based on an objective that carotid body ablation will result in a clinically significant or clinically beneficial change in the patient's physiological or clinical course. It is reasonable to believe that if the desired clinical or physiological changes occur even in the absence of meeting the predefined screening criteria, then therapy could be performed.

While the invention has been described in connection with what is presently considered to be the best mode, it is to be understood that the invention is not to be limited to the disclosed embodiment (s). The invention covers various modifications and equivalent arrangements included within the scope of the appended claims.

## Claims

1. An endovascular carotid body ablation catheter (103), configured to be inserted through a patient's vasculature to a common carotid artery (102) and to ablate a carotid body (101) or associated tissue, the endovascular carotid body ablation catheter (103) comprising:
a distal and a proximal end;
a handle (104) which remains outside of the body; and
a bipolar electrode arrangement having an active and a return electrode placed on separate shafts of the endovascular carotid body ablation catheter (103) configured to create a lesion to ablate the carotid body (101) or associated tissue.

2. A system comprising:
the endovascular carotid body ablation catheter (103) of claim 1;
an energy generator or an energy supply (210) to provide energy to the bipolar electrode arrangement; and
a computer controller (110) to automatically adjust frequency and strength of the energy applied to the catheter, timing and period during which energy is applied, and safety limits to the application of energy for ablation of a carotid body (101).

3. The endovascular carotid body ablation catheter (103) of claim 1, wherein the bipolar electrode arrangement is configured to contact a vessel wall of an external or internal carotid artery proximate the carotid body (101).

4. The endovascular carotid body ablation catheter of claim 3, wherein the ablation catheter further comprises an expandable device at the distal end of the catheter (103).

5. The endovascular carotid body ablation catheter of claim 4, wherein the expandable device includes an inflatable balloon.

6. The endovascular carotid body ablation catheter of claim 5, wherein the balloon is configured to cool tissue surrounding the lesion during an ablation procedure.

7. The endovascular carotid body ablation catheter of claim 4, wherein the expandable device includes an expandable filter such a mesh or a frame or a wire-type expandable device such as a basket or an umbrella.

8. The endovascular carotid body ablation catheter of anyone of claims 1 or 7, wherein the RF electrodes are equal or less than 3 mm in diameter and less than 5 mm in length.

## Patentansprüche

1. Endovaskulärer Katheter zur Ablation eines Glomus caroticus (103), der dazu ausgelegt ist, durch Gefäße eines Patienten an eine gemeinsamen Carotide (102) eingeführt zu werden und einen Glomus caroticus (101) oder zugehöriges Gewebe zu ablatieren, wobei der endovaskuläre Katheter zur Ablation eines Glomus caroticus (103) umfasst:
ein distales und ein proximales Ende;
einen Griff (104), der außerhalb des Körpers verbleibt; und
eine bipolare Elektrodenanordnung mit einer aktiven und einer Rückführ-Elektrode, die auf separaten Schäften des endovaskulären Katheters zur Ablation eines Glomus caroticus (103) platziert sind, um eine Läsion zu erzeugen zur Ablation des Glomus caroticus (101) oder zugehörigen Gewebes.

2. System, das umfasst:
den endovaskulärer Katheter zur Ablation eines Glomus caroticus (103) gemäß Anspruch 1;
einen Energie-Generator oder eine Energie-Versorgung (210) zur Bereitstellung von Energie an die bipolare Elektrodenanordnung; und
eine Computer-Steuerung (110) zum automatischen Einstellen von Frequenz und Stärke der Energie, die dem Katheter zugeführt wird, zeitlicher Abfolge und Dauer, während der die Energie zugeführt wird, und Sicherheitslimits für die Zuführung von Energie zur Ablation des Glomus caroticus (101).

3. Endovoskulärer Katheter zur Ablation eines Glomus caroticus (103) gemäß Anspruch 1, wobei die bipolare Elektrodenanordnung dazu ausgelegt ist, eine Gefäßwand einer äußeren oder inneren Carotide in der Nähe des Glomus caroticus (101) zu kontaktieren.

4. Endovosculärer Katheter zur Ablation eines Glomus caroticus (103) gemäß Anspruch 3, wobei der Ablationskatheter weiter eine expandierbare Vorrichtung an dem distalen Ende des Katheters (103) umfasst.

5. Endovoskulärer Katheter zur Ablation eines Glomus caroticus (103) gemäß Anspruch 4, wobei die expandierbare Vorrichtung einen aufblasbaren Ballon beinhaltet.

6. Endovoskulärer Katheter zur Ablation eines Glomus caroticus (103) gemäß Anspruch 5, wobei der Ballon dazu ausgelegt ist, Gewebe, das die Läsion umgibt, während des Ablationsvorgangs zu kühlen.

7. Endovoskulärer Katheter zur Ablation eines Glomus caroticus (103) gemäß Anspruch 4, wobei die expandierbare Vorrichtung einen expandierbaren Filter beinhaltet, wie ein Netz oder Rahmen oder eine expandierbare Vorrichtung, wie einen Korb oder einen Schirm.

8. Endovoskulärer Katheter zur Ablation eines Glomus caroticus (103) gemäß irgendeinem der Ansprüche 1 oder 7, wobei die RF-Elektroden einen Durchmesser von weniger oder gleich 3 mm und eine Länge von unter 5 mm aufweisen.

## Revendications

1. Cathéter d'ablation de corpuscule carotidien endovasculaire (103), configuré pour être inséré à travers le système vasculaire d'un patient jusqu'à une artère carotide commune (102) et pour procéder à l'ablation d'un corpuscule carotidien (101) ou d'un tissu associé, le cathéter d'ablation de corpuscule carotidien endovasculaire (103) comprenant :
une extrémité distale et une extrémité proximale ;
une poignée (104) qui reste en dehors du corps ; et
un agencement d'électrode bipolaire, ayant une électrode active et une électrode de retour placées sur des arbres séparés du cathéter d'ablation de corpuscule carotidien endovasculaire (103), configuré pour créer une lésion pour procéder à l'ablation du corpuscule carotidien (101) ou d'un tissu associé.

2. Système comprenant :
le cathéter d'ablation de corpuscule carotidien endovasculaire (103) de la revendication 1 ;
un générateur d'énergie ou une alimentation en énergie (210) pour fournir de l'énergie à l'agencement d'électrode bipolaire, et
un contrôleur informatique (110) pour ajuster automatiquement la fréquence et l'intensité de l'énergie appliquée au cathéter, la synchronisation et la période au cours de laquelle l'énergie est appliquée, et des limites de sécurité à l'application de l'énergie pour l'ablation d'un corpuscule carotidien (101).

3. Cathéter d'ablation de corpuscule carotidien endovasculaire (103) de la revendication 1, dans lequel l'agencement d'électrode bipolaire est configuré pour être au contact d'une paroi vasculaire d'une artère carotide externe ou interne à proximité du corpuscule carotidien (101).

4. Cathéter d'ablation de corpuscule carotidien endovasculaire de la revendication 3, dans lequel le cathéter d'ablation comprend un dispositif extensible au niveau de l'extrémité distale du cathéter (103).

5. Cathéter d'ablation de corpuscule carotidien endovasculaire de la revendication 4, dans lequel le dispositif extensible comprend un ballon gonflable.

6. Cathéter d'ablation de corpuscule carotidien endovasculaire de la revendication 5, dans lequel le ballon est configuré pour refroidir les tissus entourant la lésion au cours d'une procédure d'ablation.

7. Cathéter d'ablation de corpuscule carotidien endovasculaire de la revendication 4, dans lequel le dispositif extensible comprend un filtre extensible, tel qu'un maillage ou une trame ou un dispositif extensible de type câble, tel qu'un panier ou un parapluie.

8. Cathéter d'ablation de corpuscule carotidien endovasculaire de l'une quelconque des revendications 1 ou 7, dans lequel les électrodes RF ont un diamètre inférieur ou égal à 3 mm, et une longueur inférieure à 5 mm.
